# EUROPÄISCHE PATENTSCHRIFT

(11) **EP 2 915 580 B1**
(45) Veröffentlichungstag und Bekanntmachung des Hinweises auf die Patenterteilung: **29.04.2020**
(21) Anmeldenummer: 14158336.9
(22) Anmeldetag: 07.03.2014
(51) Int. Cl.: B01F 17/00, A61Q 13/00

(54) **Zubereitungen mit verbesserten physikalischen Eigenschaften**
Preparations with improved physical properties
Préparations aux propriétés physiques améliorées

(43) Veröffentlichungstag der Anmeldung: 09.09.2015
(73) Patentinhaber: Symrise AG, 37603 Holzminden (DE)
(72) Erfinder: Finke, Anja, 37603 Holzminden (DE); Lange, Sabine, 37603 Holzminden (DE); Schmaus, Gerhard, 37671 Höxter-Bosseborn (DE)
(74) Vertreter: Eisenführ Speiser

(56) Entgegenhaltungen:
- EP-A1- 2 316 408
- US-A1- 2003 007 986
- US-A1- 2009 238 787

## Beschreibung

Die vorliegende Erfindung betrifft eine Zubereitung mit verbesserten physikalischen Eigenschaften.

Sofern es sich bei der erfindungsgemäßen Zubereitung um eine O/W-Emulsion (Öl-in-Wasser-Emulsion, d.h. Öltröpfchen in Wasser) handelt, besitzt diese eine Partikelgrößenverteilung, für die gilt: D(v, 0.9) = 40 µm oder weniger. Vorzugsweise liegt die Tröpfchengröße in einem Bereich bis 40 µm, vorzugsweise in einem Bereich bis 30 µm. Die Zubereitung ist dabei aufgrund der Anwesenheit von einem oder mehreren Hydroxyestern der Formel (I) 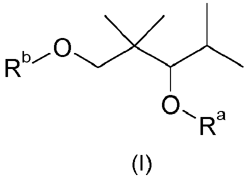 (zur Bedeutung der Substituenten R^{b} und R^{a} siehe weiter unten) so eingestellt, dass die O/W-Emulsion im Vergleich mit einer Vergleichsemulsion, die bei ansonsten gleicher Zusammensetzung keinen Hydroxyester der Formel (I) umfasst, eine erhöhte Emulsionsstabilität besitzt.

Sofern die erfindungsgemäße Zubereitung eine (vorzugsweise einphasige) tensidhaltige Zubereitung ist, ist die Gesamtmenge an einem oder mehreren Hydroxyestern der Formel (I) (zur Bedeutung der Substituenten R^{b} und R^{a} siehe wieder weiter unten) in der Zubereitung, enthaltend ein oder mehrere anionische Tenside, so gewählt, dass die tensidhaltige Zubereitung im Vergleich mit einer Vergleichszubereitung, die bei ansonsten gleicher Zusammensetzung keinen Hydroxyester der Formel (I) umfasst, bei 22,7 °C eine erhöhte Viskosität besitzt.

Bei der erfindungsgemäßen Zubereitung kann es sich um eine tensidhaltige O/W-Emulsion handeln. Sofern es sich um eine solche tensidhaltige O/W-Emulsion handelt, ist aufgrund der genannten Anwesenheit von Hydroxyestern der Formel (I) im angegebenen Sinne die Emulsionsstabilität erhöht und/oder die Viskosität erhöht.

Die vorliegende Erfindung betrifft zudem eine O/W-Emulsion umfassend als Emulgator ein oder mehrere Hydroxyester der obigen Formel (I), mit einer Partikelgrößenverteilung für die gilt:
D(v, 0.9) = 40 µm oder weniger, vorzugsweise D(v, 0.9) = 30 µm oder weniger,
D (v, 0.5) = 20 µm oder weniger, vorzugsweise D (v, 0.5) < 15 µm oder weniger.

Die vorliegende Erfindung betrifft zudem eine O/W-Emulsion umfassend als Emulgator einen oder mehrere Hydroxyester der obigen Formel (I), wobei die Stabilität der Emulsion so eingestellt ist, dass nach einer Lagerung dieser Emulsion von 3 Wochen (21 Tage) bei 25 °C und einem Druck von 1013 HPa unter Luftabschluss für die Partikelgrößenverteilung gilt: D(v, 0.9) (t_{21d}) : D(v, 0.9) (t_{0d}) < 2, bevorzugt < 1,5, besonders bevorzugt < 1,2 (D.h. der Quotient aus D(v, 0.9) nach 21 Tagen (Zähler) und D(v, 0.9) zum Startzeitpunkt der Lagerung (Nenner) ist < 2, bevorzugt < 1,5, besonders bevorzugt < 1,2).

Die vorliegende Erfindung betrifft zudem eine (vorzugsweise einphasige) tensidhaltige Zubereitung umfassend als Mittel zur Erhöhung der Viskosität bei 22,7 °C ein oder mehrere Hydroxyester der obigen Formel (I), sowie ein oder mehrere anionische Tenside, die vorzugsweise ausgewählt sind aus der Gruppe bestehend aus Sulfaten und Sulfonaten, besonders bevorzugt umfassend Natriumlaurylethersulfat sowie gegebenenfalls ein oder mehr weitere Tenside.

Die Erfindung betrifft auch die Verwendung (a) eines einzelnen Hydroxyesters der Formel (I) oder (b) einer Mischung umfassend oder bestehend aus zwei oder mehr unterschiedlichen Hydroxyestern der obigen Formel (I), als Mittel zur Modifizierung einer oder mehrerer physikalischer Eigenschaften einer Zubereitung, wobei die physikalischen Eigenschaften ausgewählt sind aus der Gruppe bestehend aus Emulsionsstabilität und Viskosität. Im Rahmen der vorliegenden Erfindung umfassen Zubereitungen insbesondere medizinische und kosmetische Formulierungen einschließlich insbesondere Körperpflegeprodukten (beispielsweise kosmetische Präparate wie Haarfärbe-, Haarverformungs- oder Enthaarungsmittel), sowie Haushalts- und (tensidhaltige) Reinigungsprodukte.

Schließlich betrifft die vorliegende Erfindung auch ein Verfahren zum Herstellen und/oder Stabilisieren einer O/W-Emulsion, mit folgenden Schritten:
Vermengen zweier (unter den gegebenen Bedingungen nicht vollständig mischbaren) Flüssigkeiten in Gegenwart einer emulgierend wirkenden Menge von einem, zwei oder mehr Hydroxyestern der obigen Formel (I).

Die vorliegende Erfindung betrifft ferner auch noch ein Verfahren zum Herstellen einer tensidhaltigen Zubereitung mit erhöhter Viskosität, enthaltend ein oder mehrere anionische Tenside, mit folgenden Schritten:
Vermengen einer tensidhaltigen Flüssigkeit, enthaltend ein oder mehrere anionische Tenside, mit einer viskositätserhöhenden Menge von einem, zwei oder mehr Hydroxyestern der obigen Formel (I).

Es besteht ein ständiger Bedarf an chemischen Zubereitungen, insbesondere kosmetischen und medizinischen Zubereitungen, deren physikalische Eigenschaften spezifisch eingestellt sind. Dementsprechend besteht auch ein ständiger Bedarf an Hilfsstoffen, welche in der Lage sind, Basis-Zubereitungen durch ihre Zugabe so zu modifizieren, dass die erwünschten physikalischen Eigenschaften erreicht werden.

Beispielsweise besteht ein ständiger Bedarf an neuen O/W-Emulsionen und tensidhaltigen Zubereitungen mit maßgeschneiderten physikalischen Eigenschaften. Bei O/W-Emulsionen, insbesondere bei solchen O/W-Emulsionen, die als kosmetische oder medizinische Zubereitung vorgesehen sind, wird häufig darauf Wert gelegt, dass diese Emulsionen besonders weiß erscheinen.

Im Bereich der tensidhaltigen Zubereitungen besteht häufig der Wunsch, dass diese eine vergleichsweise hohe Viskosität besitzen sollen, insbesondere wenn es sich um eine tensidhaltige (Reinigungs-) Zubereitung für kosmetische Zwecke handelt.

In eigenen Untersuchungen betreffend O/W-Emulsionen wurde davon ausgegangen, dass der Weißegrad einer O/W-Emulsionen maßgeblich davon abhängt, welche Partikelgrößenverteilung die emulgierten Öl-Partikel innerhalb der wässrigen kontinuierlichen Phase besitzen. Ein höherer Weißegrad ergibt sich demnach immer dann, wenn eine Vielzahl der emulgierten Partikel eine besonders geringe Partikelgröße besitzt. Akzeptable Weißegrade wurden in eigenen Untersuchungen regelmäßig dann erreicht, wenn für die Partikelgrößenverteilung in der O/W-Emulsion gilt: D(v, 0.9) = 40 µm oder weniger, vorzugsweise 30 µm oder weniger. Vorzugsweise liegt die Tröpfchengröße in einem Bereich bis 40 µm, vorzugsweise im Bereich bis 30 µm. Es versteht sich hierbei, dass es bei der Konzeption und Entwicklung einer neuen O/W-Emulsion nicht nur darauf ankommt, einmalig oder nur anfänglich eine Partikelgrößenverteilung für die emulgierten Partikel einzustellen, welche einen hohen Weißegrad vermittelt, sondern gleichzeitig dafür zu sorgen, dass diese Partikelgrößenverteilung auch über einen langen Lagerzeitraum stabil erhalten bleibt.

Es war eine Aufgabe der vorliegenden Erfindung, Zubereitungen, nämlich O/W-Emulsionen und tensidhaltige Zubereitungen anzugeben, deren physikalische Eigenschaften besonders günstig eingestellt sind. Hinsichtlich der O/W-Emulsionen war es dabei gemäß einem Teilaspekt der vorliegenden Erfindung anzustreben, eine besonders stabile Emulsion mit hohem Weißegrad anzugeben. Hinsichtlich der tensidhaltigen Zubereitungen war es gemäß einem weiteren Teilaspekt der vorliegenden Erfindung anzustreben, bei 22,7 °C eine vergleichsweise hohe Viskosität zu erreichen.

Es hat sich in eigenen Untersuchungen gezeigt, dass sich die vorstehend genannte Aufgabe (auch hinsichtlich der genannten Teilaspekte) unter Einsatz derselben chemischen Hilfsmittel lösen lässt.

Gemäß einem ersten Aspekt der vorliegenden Erfindung wird die gestellte Aufgabe gelöst durch eine Zubereitung umfassend
- einen oder mehrere Hydroxyester der Formel (I) 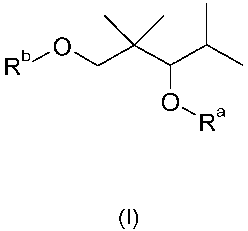
   wobei jeweils einer der beiden Reste R^{a} oder R^{b} Wasserstoff und der jeweils andere Rest R^{a} oder R^{b} einen geradkettigen oder verzweigten Acylrest mit 2 bis 10 C-Atomen bedeutet,
   sowie
- ein oder mehrere weitere Verbindungen,
   wobei die Zubereitung eine O/W-Emulsion ist, für deren Partikelgrößenverteilung gilt: D(v, 0.9) = 40 µm oder weniger, wobei in der Zubereitung die Gesamtmenge an dem oder den Hydroxyestern der Formel (I) so gewählt ist, dass die O/W-Emulsion im Vergleich mit einer Vergleichsemulsion, die bei ansonsten gleicher Zusammensetzung keinen Hydroxyester der Formel (I) umfasst, eine erhöhte Emulsionsstabilität besitzt
   und/oder
   wobei die Zubereitung eine (vorzugsweise einphasige) tensidhaltige Zubereitung ist, enthaltend ein oder mehrere anionische Tenside, und die Gesamtmenge an dem oder den Hydroxyestern der Formel (I) in der Zubereitung so gewählt ist, dass die Zubereitung im Vergleich mit einer Vergleichszubereitung, die bei ansonsten gleicher Zusammensetzung keinen Hydroxyester der Formel (I) umfasst, bei 22,7 °C eine erhöhte Viskosität besitzt.

Vorzugsweise gilt für die Partikelgrößenverteilung der oben genannten O/W-Emulsionen: D(v, 0.9) = 30 µm oder weniger. Besonders bevorzugt liegt die Tröpfchengröße (in einer wie ansonsten oben definierten O/W-Emulsion) in einem Bereich bis 40 µm, vorzugsweise in einem Bereich bis 30 µm.

Besonders bevorzugt ist eine Zubereitung (wie vorstehend definiert), wobei R^{a} des einen Hydroxyesters der Formel (I) Wasserstoff bedeutet, bzw. wobei R^{a} (i) eines einzelnen Hydroxyesters der Formel (I) (von insgesamt mehreren Hydroxyestern der Formel (I)) oder (ii) bei zwei, drei oder mehr als drei Hydroxyestern dieser insgesamt mehreren Hydroxyester der Formel (I) oder (iii) bei sämtlichen dieser insgesamt mehreren Hydroxyester der Formel (I) Wasserstoff bedeutet.

Ganz besonders bevorzugt ist eine Zubereitung (wie vorstehend definiert, insbesondere wie vorstehend als bevorzugt definiert) umfassend mindestens einen Hydroxyester der Formel (I), wobei R^{a} Wasserstoff bedeutet, vorzugsweise R^{a} Wasserstoff bedeutet und R^{b} einen geradkettigen oder verzweigten Acylrest mit 3 bis 6 Kohlenstoffatomen bedeutet (die vorstehend genannten und bevorzugten Bedeutungen zu R^{a} und R^{b} gelten ganz besonders bevorzugt für Zubereitungen, die O/W-Emulsionen sind).

Verbindungen der obigen Formel (I) wurden bereits in der Veröffentlichung EP 2 133 102 A1 unter Verweis auf eine Vielzahl weiterer Dokumente aus dem Stand der Technik offenbart und diskutiert. Gemäß EP 2 133 102 A1 sind Verbindungen der Formel (I) in hervorragender Weise zur Reduzierung von Gerüchen geeignet.

Verbindungen der Formel (I) sind Monoester des 2,2,4-Trimethylpentan-1,3-diols. Solche Verbindungen der Formel (I) sind teilweise bereits seit längerer Zeit kommerziell verfügbar (vgl. US 3,329,713) beziehungsweise können auf bekannte Weise durch Veresterung von 2,2,4-Trimethylpentan-1,3-diol erhalten werden, wie beispielsweise in Liebigs Ann. 1972, 756, 162-169 oder US 3,408,388 beschrieben. Alternative Verfahren zur Herstellung der Verbindungen der Formel (I) sind beispielsweise aus US 3,091,632, DE 3403696, US 5,166,413, WO 98/24752 oder DE 10207747 sowie der jeweils darin zitierten Literatur bekannt bzw. können in Anlehnung an diese durchgeführt werden.

Überraschenderweise hat sich in eigenen Untersuchungen gezeigt, dass die Hydroxyester der Formel (I) in der Lage sind, die physikalischen Eigenschaften von chemischen Zubereitungen (insbesondere kosmetischen und medizinischen Zubereitungen) in der vorstehend geschilderten gewünschten Weise zu beeinflussen. Hydroxyester der Formel (I) stabilisieren O/W-Emulsionen und insbesondere O/W-Emulsionen, für deren Partikelgrößenverteilung gilt: D(v, 0.9) = 40 µm oder weniger, vorzugsweise D(v, 0.9) = 30 µm oder weniger. Zudem erhöhen Verbindungen der Formel (I) regelmäßig die Viskosität von tensidhaltigen Zubereitungen (soweit nachfolgend nicht anders angegeben, wird die Viskosität bei 22,7 °C bestimmt).

Es hat sich in eigenen Untersuchungen darüber hinaus gezeigt, dass die erfindungsgemäßen Zubereitungen bzw. die darin enthaltenen Hydroxyester der Formel (I) regelmäßig eine, mehrere oder sämtliche der folgenden Sekundäreigenschaften aufweisen:
- leichte Zugänglichkeit,
- Einsetzbarkeit auch in konzentrierter Form,
- weitgehende oder vollständige Farblosigkeit,
- hohe Stabilität innerhalb der jeweiligen Zubereitung
- inertes Verhalten (z.B. gegenüber den anderen Zubereitungsbestandteilen),
- keine relevante toxische und/oder allergene Wirkung gegenüber dem Menschen,
- geringer Eigengeruch.

Erfindungsgemäße Zubereitungen können zusätzlich beispielsweise Hilfsstoffe umfassen, wie zum Beispiel Dipropylenglykol (DPG), Diethylphthalat (DEP), Triethylcitrat (TEC), Isopropylmyristat (IPM) und Benzylbenzoat (BB).

Im Zusammenhang mit der vorliegenden Erfindung wurde die Partikelgrößenverteilung mittels des Prinzips der Laserbeugung bestimmt. Entsprechende Geräte zur Bestimmung der Partikelgröße bzw. der Partikelgrößenverteilung werden von der Malvern Instruments GmbH und der Malvern Instruments Ltd. vertrieben. Sofern nachfolgend nicht anders angegeben, werden Partikelgrößenverteilungen mittels eines Gerätes des Typs Malvern Mastersizer Micro MAF 5000 bestimmt. Bei der Bestimmung der Partikelgrößenverteilung mittels Laserbeugung wird die Intensität des gestreuten Lichts eines Laserstrahls gemessen, während dieser eine Probe einer zu untersuchenden O/W-Emulsion durchdringt. Aus den erhaltenen Daten wird die Größe der Partikel und die zugehörige Partikelgrößenverteilung aus dem erzeugten Beugungsmuster berechnet.

Die Viskosität einer erfindungsgemäßen tensidhaltigen (vorzugsweise einphasigen) Zubereitung ist von der Temperatur abhängig. Sofern nachfolgend nicht anders angegeben, werden im Rahmen der vorliegenden Erfindung Viskositäten von unterschiedlichen tensidhaltigen Zubereitungen bei einer Temperatur von 22,7 °C bestimmt, um diese vergleichen zu können. Sofern nicht anders angegeben, wird im Rahmen der vorliegenden Erfindung die Viskosität bei der definierten Temperatur mittels eines Viskosimeters bestimmt, welches nach dem Rotations-Prinzip arbeitet. Der Fachmann setzt beispielsweise zur Bestimmung der Viskosität ein Viskosimeter der Firma Haake (Haake Viscotester 7 plus) ein.

Bevorzugt ist eine erfindungsgemäße Zubereitung (wie oben definiert, vorzugsweise wie oben als bevorzugt definiert) umfassend
- einen oder mehrere Hydroxyester der Formel (I) 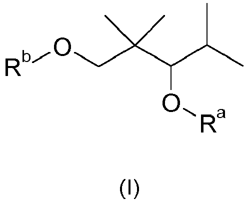 wobei jeweils einer der beiden Reste R^{a} oder R^{b} Wasserstoff und der jeweils andere Rest R^{a} oder R^{b} einen geradkettigen oder verzweigten Acylrest mit 2 bis 6 C-Atomen bedeutet,
wobei die Zubereitung vorzugsweise eine solche O/W-Emulsion ist, für deren Partikelgrößenverteilung gilt: D(v, 0.9) = 40 µm oder weniger, und bei der die Gesamtmenge an diesem oder diesen Hydroxyestern der Formel (I) mit einem Acylrest mit 2 bis 6 C-Atomen in der Zubereitung so gewählt ist, dass die O/W-Emulsion im Vergleich mit einer Vergleichsemulsion, die bei ansonsten gleicher Zusammensetzung keinen solchen Hydroxyester der Formel (I) mit einem Acylrest mit 2 bis 6 C-Atomen umfasst, eine erhöhte Emulsionsstabilität besitzt
und/oder
wobei die Zubereitung vorzugsweise eine solche tensidhaltige Zubereitung ist (besonders bevorzugt ist eine einphasige tensidhaltige Zubereitung), bei der die Gesamtmenge an diesem oder diesen Hydroxyester der Formel (I) mit einem Acylrest mit 2 bis 6 C-Atomen in der Zubereitung so gewählt ist, dass die Zubereitung im Vergleich mit einer Vergleichszubereitung, die bei ansonsten gleicher Zusammensetzung keinen solchen Hydroxyester der Formel (I) mit einem Acylrest mit 2 bis 6 C-Atomen umfasst, bei 22,7 °C eine erhöhte Viskosität besitzt.

In den vorstehend genannten bevorzugten Ausgestaltungen einer erfindungsgemäßen Zubereitung kommt es also entscheidend auf die Anwesenheit solcher Hydroxyester der Formel (I) an, die einen Acylrest mit zwei bis sechs C-Atomen besitzen. Eine erhöhte Emulsionsstabilität beziehungsweise eine erhöhte Viskosität (jeweils im Vergleich mit einer entsprechenden Vergleichszubereitung) liegt vor im Vergleich mit Vergleichszubereitungen, die keinen solchen Hydroxyester der Formel (I) mit einem Acylrest mit zwei bis sechs C-Atomen umfassen, unabhängig von der Anwesenheit von Hydroxyestern der Formel (I) mit einem Alkylrest mit sieben bis zehn C-Atomen. Es hat sich in eigenen Untersuchungen gezeigt, dass die Hydroxyester der Formel (I) mit einem Acylrest mit zwei bis sechs C-Atomen regelmäßig besonders wirksam sind hinsichtlich der vorteilhaften Einstellung der oben genannten physikalischen Eigenschaften.

Besonders bevorzugt ist eine erfindungsgemäße Zubereitung (wie vorstehend definiert), wobei der Acylrest eines, zweier, mehrerer oder sämtlicher der Hydroxyester der Formel (I) ausgewählt ist aus der Gruppe bestehend aus Acetyl, Propionyl, n-Butyryl, Isobutyryl, Crotonyl, n-Pentanoyl, Isopentanoyl, n-Hexanoyl und Isohexanoyl, vorzugsweise ausgewählt aus der Gruppe bestehend aus n-Butyryl, Isobutyryl und Crotonyl.

Bevorzugte Acylreste als Rest R^{b} bzw. R^{a} eines, zweier, mehrerer oder sämtlicher (bevorzugt eingesetzter) Hydroxyester der Formel (I) sind die in der folgenden Liste auch durch Formelbilder spezifizierten Acylreste, wobei in der jeweiligen Strukturformel die gestrichelte Linie eine kovalente Bindung bedeutet zwischen dem gezeichneten Acylrest (Strukturformel) und dem benachbarten Sauerstoffatom des Hydroxyesters der Formel (I).

| | **Formel** | **Strukturformel** |
|---|---|---|
| Acetyl | **-C(O)-CH₃** | 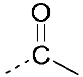 |
| Propionyl | **-C(O)-CH₂-CH₃** | 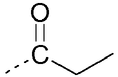 |
| n-Butyryl | **-C(O)-CH₂-CH₂-CH₃** | 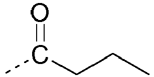 |
| Isobutyryl | **-C(O)-CH(CH₃)₂** | 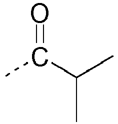 |
| Crotonyl | **-C(O)-CH=CH-CH₃** | 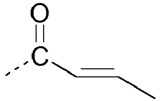 |
| n-Pentanoyl | **-C(O)-CH₂-CH₂-CH₂-CH₃** | 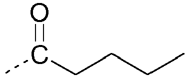 |
| Isopentanoyl | **-C(O)-CH₂-CH(CH₃)₂** | 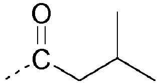 |
| | **-C(O)-CH(CH₃)-CH₂-CH₃** | 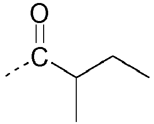 |
| | **-C(O)-C(CH₃)₃** | 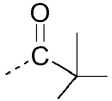 |
| n-Hexanoyl | **-C(O)-(CH₂)₄-CH₃** | 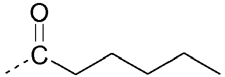 |
| Isohexanoyl | **-C(O)-(CH₂)₂-CH(CH₃)₂** | 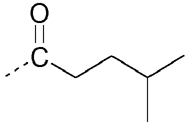 |
| | **-C(O)-(CH₂)-CH(CH₃)-CH₂-CH₃** | 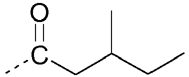 |
| | **-C(O)-CH(CH₃)-(CH₂)₂-CH₃** | 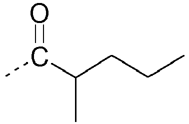 |
| | **-C(O)-CH(CH₃)-CH(CH₃)₂** | 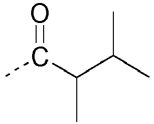 |
| | **-C(O)-C(CH₃)₂-CH₂-CH₃** | 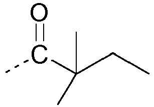 |
| | **-C(O)-CH₂-C(CH₃)₃** | 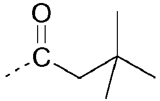 |

Eine erfindungsgemäße Zubereitung ist häufig bevorzugt, wenn sie einen ersten Hydroxyester der Formel (I) wie vorstehend definiert (insbesondere wie vorstehend als bevorzugt definiert) umfasst, wobei der Rest R^{a} Wasserstoff und der Rest R^{b} einen geradkettigen oder verzweigten Acylrest (vorzugsweise einen Rest R^{b} wie vorstehend gelistet) bedeutet.

Entsprechend umfasst eine erfindungsgemäße Zubereitung vorzugsweise einen ersten Hydroxyester der Formel (I) (wie vorstehend definiert, insbesondere wie vorstehend als bevorzugt definiert, vorzugsweise wie aufgelistet), wobei der Rest R^{a} Wasserstoff und der Rest R^{b} einen geradkettigen oder verzweigten Acylrest bedeutet sowie gegebenenfalls zusätzlich einen zweiten Hydroxyester der Formel (I) (wie vorstehend definiert, insbesondere wie vorstehend als bevorzugt definiert), wobei der Rest R^{b} Wasserstoff und der Rest R^{a} einen geradkettigen oder verzweigten Acylrest bedeutet, wobei dieser Acylrest R^{a} des zweiten Hydroxyesters die gleiche Struktur wie der Acylrest R^{b} des ersten Hydroxyesters hat,
wobei die Zubereitung vorzugsweise eine O/W-Emulsion ist, für deren Partikelgrößenverteilung gilt: D(v, 0.9) = 40 µm oder weniger, wobei die Gesamtmenge an dem ersten und dem zweiten Hydroxyester der Formel (I) in der Zubereitung vorzugsweise so gewählt ist, dass die O/W-Emulsion im Vergleich mit einer Vergleichsemulsion, die bei ansonsten gleicher Zusammensetzung keinen solchen ersten und zweiten Hydroxyester der Formel (I) umfasst, eine erhöhte Emulsionsstabilität besitzt
und/oder
wobei die Gesamtmenge an dem ersten und dem zweiten Hydroxyester der Formel (I) in der (vorzugsweise einphasigen) Zubereitung vorzugsweise so gewählt ist, dass die Zubereitung im Vergleich mit einer Vergleichszubereitung, die bei ansonsten gleicher Zusammensetzung keinen solchen ersten und zweiten Hydroxyester der Formel (I) umfasst, bei 22,7 °C eine erhöhte Viskosität besitzt.

Selbstverständlich ist es auch in diesem Fall, also in dem Fall, in dem die erfindungsgemäße Zubereitung einen ersten Hydroxyester der Formel (I) (wie oben definiert, vorzugsweise wie oben als bevorzugt definiert) umfasst, wobei der Rest R^{a} Wasserstoff und der Rest R^{b} einen geradkettigen oder verzweigten Acylrest bedeutet, und einen zweiten Hydroxyester umfasst, wobei der Rest R^{b} Wasserstoff und der Rest R^{a} einen geradkettigen oder verzweigten Acylrest bedeutet, wobei dieser Acylrest R^{a} des zweiten Hydroxyesters die gleiche Struktur wie der Acylrest R^{b} des ersten Hydroxyesters hat bevorzugt, wenn der jeweilige Acylrest zwei bis sechs C-Atome aufweist (vorzugsweise ist der Acylrest wieder ausgewählt aus der obigen Liste). Ganz besonders bevorzugt ist es erneut, wenn der Acylrest eines, zweier, mehrerer oder sämtlicher der Hydroxyester der Formel (I), in denen der Rest R^{a} Wasserstoff und der Rest R^{b} einen geradkettigen oder verzweigten Acylrest bedeutet, ausgewählt ist aus der vorstehend bereits genannten Gruppe bestehend aus Acetyl, Propionyl, n-Butyryl, Isobutyryl, Crotonyl, n-Pentanoyl, Isopentanoyl, n-Hexanoyl und Isohexanoyl,
vorzugsweise ausgewählt aus der Gruppe bestehend aus n-Butyryl, Isobutyryl und Crotonyl.

Gemäß einem Aspekt der vorliegenden Erfindung sind erfindungsgemäße Zubereitungen (wie vorstehend definiert, insbesondere wie vorstehend als bevorzugt definiert) bevorzugt, wobei einer, zwei, mehrere oder sämtliche der Hydroxyester der Formel (I) (wie oben beschrieben, vorzugsweise wie oben als bevorzugt definiert) einen verzweigten Acylrest besitzen.

Für manche Anwendungen ist alternativ eine erfindungsgemäße Zubereitung bevorzugt, wobei einer, zwei, mehrere oder sämtliche der Hydroxyester der Formel (I) (wie oben beschrieben, vorzugsweise wie oben als bevorzugt definiert) einen unverzweigten Acylrest besitzen.

Besonders bevorzugt ist eine erfindungsgemäße Zubereitung (wie vorstehend definiert, vorzugsweise wie oben als bevorzugt definiert), wobei der erste Hydroxyester der Formel (I) die Formel 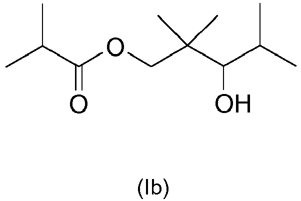 besitzt
und der gegebenenfalls vorhandene zweite Hydroxyester der Formel (I) die Formel 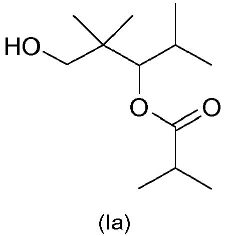 besitzt.

Die Verbindung der Formel (la) (CAS-Nummer 18491-15-1) und die Verbindung der Formel (Ib) (CAS-Nummer 77-68-9) sind jeweils an sich bereits bekannt. Ebenso sind Mischungen aus Verbindung (la) und Verbindung (Ib) bekannt (CAS-Nummer 25265-77-4). Solche Mischungen sind kommerziell erhältlich, beispielsweise von den Firmen BASF, DSM, Eastman oder Perstorp.

Yukagaku (1993), 42(1), S. 44-48 offenbart das Vorkommen von 2-Methylpropionsäure-3-hydroxy-2,2,4-trimethylpentylester der obigen Formel (Ib) in bestimmten Pflanzen der Familie Polypodiaceae. Dabei wurden auch die Riechstoffe Hexanal, Heptanal, 4-Hexen-1-ol, 1-Hepten-3-ol, Vanillin, Cedrol, Linalool, beta-Ionon, alpha-Pinen, Isobutylisobutyrat, alpha-Terpineol und Ethylcinnamat gefunden. Die offenbarten Mischungen sind nicht Gegenstand der vorliegenden Erfindung. Es handelt sich bei den offenbarten Mischungen nicht um Zubereitungen im Sinne der obigen Definition.

Yukagaku (1989), 38(9), S. 689-693, Nippon Nogei Kagaku Kaishi (1989), 63(7), S. 1231-1234 und Nippon Nogei Kagaku Kaishi (1988), 62(12), S. 1763-1768 berichten über das Vorkommen von 2-Methylpropionsäure-3-hydroxy-2,2,4-trimethylpentylester der obenstehenden Formel (Ib) in verschiedenen Bohnensorten. Dabei wurden unter anderem auch Riechstoffe wie Hexanal, 2-Phenylethanol, Vanillin, Maltol, Guaiacol, 4-Vinylguiacaol, Furfurylalkohol, Furfural, Cedrol, Butylbutyrat und Ethylcinnamat identifiziert. Die offenbarten Mischungen sind nicht Gegenstand der vorliegenden Erfindung. Es handelt sich bei den offenbarten Mischungen nicht um Zubereitungen im Sinne der obigen Definition.

In JP 11222574 wird die Verwendung bestimmter auch erfindungsgemäß einzusetzender Verbindungen als Bestandteil in einem Wasser-basierten Versiegelungsmittel für poröse Baustoffe beschrieben. In JP 2003171635 wird die Verwendung bestimmter auch erfindungsgemäß einzusetzender Verbindungen als Bestandteil in einem Klebstoff für Baustoffe beschrieben. Die vorliegende Erfindung betrifft jedoch keine dieser offenbarten Verwendungen und Mischungen. Die Verwendung der erfindungsgemäß zu verwendenden Verbindungen der Formel (I) als Bestandteil eines Klebstoffs beziehungsweise eines Versiegelungsmittels für (poröse) Baustoffe ist aber im Rahmen der vorliegenden Erfindung generell nicht bevorzugt, insbesondere die Verwendung als Bestandteil in einem Wasser-basierten Versiegelungsmittel für poröse Baustoffe gemäß JP 11222574 oder als Bestandteil in einem Klebstoff für Baustoffe nach JP 2003171635.

In FR 2867972 wird die Verwendung bestimmter erfindungsgemäß einzusetzender Verbindungen in Nagellack beschrieben. Ein solcher Nagellack ist nicht Gegenstand der vorliegenden Erfindung. Die Verwendung in Lacken, insbesondere in Nagellacken ist im Rahmen der vorliegenden Erfindung generell nicht bevorzugt.

In WO 2004/062363 wird die Verwendung von 2-Methylpropionsäure-3-hydroxy-2,2,4-trimethylpentylester der obenstehenden Formel (Ib) in einer Mischung zur Bildung eines bioziden Films auf einer Oberfläche beschrieben. Diese Mischung kann optional ein - dort nicht näher spezifiziertes - Parfümöl ("fragrance") enthalten. Die in WO 2004/062363 beschriebenen Mischungen, insbesondere die Mischungen gemäß Beispiel 1 von WO 2004/062363 sind nicht Gegenstand der vorliegenden Erfindung. Die Verwendung erfindungsgemäß einzusetzender Hydroxyester der Formel (I), insbesondere der Formel (Ib), in einer Mischung zur Bildung eines bioziden Films auf einer Oberfläche, insbesondere in einer solchen Mischung umfassend ein film-bildendes Mittel ("film-forming agent") ist im Rahmen der vorliegenden Erfindung generell nicht bevorzugt.

In WO 95/21606 wird eine Verbindung der Formel (I) mit R^{b} = Isobutyryl und R^{a} = H (3-Hydroxy-2,2,4-trimethylpentylisobutyrat) als Ausgangsstoff zur Herstellung des ungesättigten 2,2,4-Trimethylpent-3-en-1-yl-isobutyrat durch Dehydratation erwähnt. In dem Zusammenhang wird dort beschrieben, dass bestimmte 2,2,4-Trimethylpenten-1-yl-Ester, wie z.B. das genannte ungesättigte Isobutyrat, als Riech- und Aromastoffe verwendet werden können. Das bezeichnete Butyrat weist dabei einen citrisch-fruchtigen, grünen, blumigen Geruch auf. Die in WO 95/21606 offenbarten Mischungen enthaltend Verbindungen der Formel (I) und Verwendungen von Verbindungen der Formel (I) sind nicht Gegenstand der vorliegenden Erfindung. Mischungen umfassend 3-3-Hydroxy-2,2,4-trimethylpentylisobutyrat und 2,2,4-Trimethylpent-3-en-1-yl-isobutyrat sind im Rahmen der vorliegenden Erfindung generell nicht bevorzugt.

In US 5,942,467 wird gemäß einer dort genannten Ausführungsform die Substanz 2-Methylpropionsäure-3-hydroxy-2,2,4-trimethylpentylester der obenstehenden Formel (Ib) als Bestandteil von Bohrflüssigkeiten vorgeschlagen. Die vorliegende Erfindung betrifft keine Verwendung erfindungsgemäß einzusetzender Hydroxyester der Formel (I) als Bestandteil von Bohrflüssigkeiten, insbesondere nicht als Bestandteil von Bohrflüssigkeiten nach US 5,942,467. Die dort offenbarten Mischungen sind nicht Gegenstand der vorliegenden Erfindung.

In Food Res. Int. 2001, 34, 473-481 und J. Food Science 2002, 67, 848-854 wird über den Beitrag von 2-Methylpropionsäure-3-hydroxy-2,2-dimethyl-1-(1-methylethyl)-propylester der obenstehenden Formel (la) zu Aromen von gekochten Garnelen bzw. gekochtem Tintenfisch berichtet. Die dort offenbarten Mischungen sind nicht Gegenstand der vorliegenden Erfindung. Im Rahmen der vorliegenden Erfindung ist eine Verwendung erfindungsgemäß einzusetzender Hydroxyester der Formel (I), insbesondere der Formel (la) in Mischungen auf Basis von gekochten Garnelen beziehungsweise gekochtem Tintenfisch nicht bevorzugt.

Die zitierten Dokumente lieferten keinen Hinweis darauf, dass die erfindungsgemäß einzusetzenden Hydroxyester der Formel (I) zur Beeinflussung der wichtigen physikalischen Parameter Emulsionsstabilität und Viskosität geeignet sind.

Erfindungsgemäße Zubereitungen können Riechstoffe umfassen. Beispiele für Riechstoffe, die vorteilhaft als Bestandteil einer erfindungsgemäßen Zubereitung verwendet werden können, finden sich z.B. in S. Arctander, Perfume and Flavor Chemicals, Vol. I und II, Montclair, N. J., 1969, Selbstverlag oder H. Surburg, J. Panten, Common Fragrance and Flavor Materials, 5th Ed., Wiley-VCH, Weinheim 2006. Insbesondere sei auf solche Riechstoffe verwiesen, wie sie in dem bereits vorstehend genannten Dokument EP 2 133 102 A1 offenbart sind. Verwiesen sei in diesem Zusammenhang insbesondere auf die Absätze [0062] und [0063] des besagten Dokuments EP 2 133 102 A1. Die dort genannten Riechstoffe sind für den Einsatz in einer bevorzugten erfindungsgemäßen Zubereitung besonders vorteilhaft; die dort genannten Riechstoffe sind im Wege der Verweisung Bestandteil der vorliegenden technischen Offenbarung.

Die erfindungsgemäßen Zubereitungen (wie oben definiert, vorzugsweise wie oben als bevorzugt definiert) können auch solche sein, welche bei Abwesenheit von Hydroxyestern der Formel (I) (wie vorstehend definiert) einen unangenehmen Geruch besitzen. In solchen Zubereitungen kann die Anwesenheit eines oder mehrerer Hydroxyester der Formel (I) (wie vorstehend definiert) dazu führen, dass der besagte unangenehme Geruch reduziert wird. In solchen Fällen tritt neben einer bereits in Dokument EP 2 133 102 A1 diskutierten Geruchsreduzierung die gemäß der vorliegenden Erfindung bestehende Wirkung der Modifizierung oder Einstellung physikalischer Eigenschaften ein, nämlich der Emulsionsstabilität und/oder der Viskosität.

Selbstverständlich betrifft die vorliegende Erfindung aber auch solche Zubereitungen, die weder einen oder mehrere Riechstoffe umfassen wie sie im Dokument EP 2 33 102 A1 angegeben sind, noch einen oder mehrere Stoffe umfassen, die für einen unangenehmen Geruch im Sinne der dortigen Offenbarung verantwortlich sind.

Gemäß einem ersten Aspekt der vorliegenden Erfindung handelt es sich bei der erfindungsgemäßen Zubereitung um eine O/W-Emulsion. Eine erfindungsgemäße O/W-Emulsion ist somit vorzugsweise eine erfindungsgemäße Zubereitung wie sie vorstehend definiert ist (vorzugsweise wie vorstehend als bevorzugt definiert). Eine bevorzugte erfindungsgemäße O/W-Emulsion umfasst als Emulgator einen oder mehrere Hydroxyester der Formel (I) wie vorstehend bei der Diskussion erfindungsgemäßer Zubereitungen definiert, und sie besitzt eine Partikelgrößenverteilung, für die gilt:
D(v, 0.9) = 40 µm oder weniger, vorzugsweise D(v, 0.9) = 30 µm oder weniger,
D (v, 0.5) = 20 µm oder weniger, vorzugsweise D (v, 0.5) = 15 µm oder weniger
und vorzugsweise zusätzlich:
D (v, 0.1) = 2,5 µm oder weniger, vorzugsweise D (v, 0.1) = 2,0 µm oder weniger.

Besonders bevorzugt sind in einer erfindungsgemäßen O/W-Emulsion somit Partikelgrößenverteilungen der emulgierten Öl-Partikel, bei denen eine vergleichsweise große Zahl der Partikel einen besonders kleinen Partikeldurchmesser besitzt. Derartige O/W-Emulsionen besitzen regelmäßig einen besonders hohen Weißegrad.

Für erfindungsgemäße O/W-Emulsionen gilt hinsichtlich bevorzugter Hydroxyester der Formel (I) generell das vorstehend im Zusammenhang mit einer erfindungsgemäßen Zubereitung Gesagte entsprechend. Ganz besonders bevorzugt ist somit wiederum der Einsatz eines Hydroxyesters der Formel (Ib). Bevorzugte erfindungsgemäße O/W-Emulsionen umfassen als Emulgator einen oder mehrere Hydroxyester der Formel (I) wie vorstehend im Zusammenhang mit erfindungsgemäßen Zubereitungen definiert (wobei eine solche bevorzugte O/W-Emulsion vorzugsweise auch eine erfindungsgemäße Zubereitung wie oben definiert ist), wobei die Stabilität der Emulsion so eingestellt ist, dass nach einer Lagerung dieser Emulsion von 3 Wochen (21 Tage) bei 25 °C und einem Druck von 1013 HPa unter Luftabschluss für die Partikelgrößenverteilung gilt: D(v, 0.9) (t_{21d}) : D(v, 0.9) (t_{0d}) < 2, bevorzugt < 1,5, besonders bevorzugt < 1,2 oder wobei der Instabilitätsindex der O/W-Emulsion, bestimmt mittels eines Lumisizers der Firma LUM, 75 % oder weniger, vorzugsweise 65 % oder weniger, besonders vorzugsweise 55 % oder weniger beträgt, verglichen mit dem Instabilitätsindex einer Vergleichs-O/W-Emulsion, die bei ansonsten gleicher Zusammensetzung keinen Hydroxyester der Formel (I) umfasst. Bezüglich der Lagerung gilt das weiter oben Gesagte entsprechend; bezüglich des Instabilitätsindexes, bestimmt mittels des Lumisizers gilt das weiter unten Gesagte entsprechend.

Zur Überprüfung, ob eine Emulsion eine derartige Stabilität besitzt, wird eine Probe einer entsprechenden O/W-Emulsion bei 25 °C und einem Druck von 1013 HPa unter Luftabschluß für drei Wochen (21 Tage) gelagert. Unmittelbar vor der Lagerung und unmittelbar nach der Lagerung wird jeweils die Partikelgrößenverteilung bestimmt (zur Methode der Bestimmung der Partikelgrößenverteilung siehe oben und die Beispiele weiter unten). Es wird dann der Quotient gebildet aus den Messwerten D(v, 0.9) (t_{21d}) und D(v, 0.9) (t_{0d}). Sofern der Quotient kleiner ist als 2 (bevorzugt < 1,5 besonders bevorzugt < 1,2) besitzt die untersuchte Emulsion die entsprechende Stabilität.

Gemäß einem weiteren Aspekt der vorliegenden Erfindung handelt es sich bei der erfindungsgemäßen Zubereitung um eine erfindungsgemäße tensidhaltige Zubereitung. Eine erfindungsgemäße tensidhaltige Zubereitung ist vorzugsweise eine erfindungsgemäße Zubereitung, wie sie weiter oben allgemein diskutiert und definiert wurde. Eine bevorzugte erfindungsgemäße (vorzugsweise einphasige) tensidhaltige Zubereitung umfasst
- als Mittel zur Erhöhung der Viskosität bei 22,7 °C einen oder mehrere Hydroxyester der Formel (I) (wie vorstehend bei der Diskussion erfindungsgemäßer Zubereitungen definiert),
- ein oder mehrere anionische Tenside, vorzugsweise ausgewählt aus der Gruppe bestehend aus Sulfaten und Sulfonaten, besonders bevorzugt umfassend Natriumlaurylethersulfat
   sowie gegebenenfalls
- ein oder mehrere weitere Tenside.

Hinsichtlich bevorzugter Hydroxyester der Formel (I) gilt wiederum das vorstehend hinsichtlich der erfindungsgemäßen Zubereitungen Gesagte entsprechend. Insbesondere bevorzugt ist also insoweit erneut der Einsatz eines Hydroxyesters der Formel (Ib).

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten, im Molekül aufweisen.

Dazu zählen z. B. Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Bei den weiteren Tensiden kann es sich um nichtionische, amphotere und kationische Tenside handeln.

Als nichtionische Tenside sind beispielsweise die Umsetzungsprodukte von aliphatischen Hydroxyestern oder Alkylphenolen mit 6 bis 20 C-Atomen in der Alkylkette, die linear oder verzweigt sein kann, mit Ethylenoxid und/oder Propylenoxid geeignet. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, ethoxylierte Fettsäureamide, Alkylpolyglycoside oder Sorbitanetherester geeignet.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als kationische Tenside eignen sich quaternäre Ammoniumverbindungen, beispielsweise Cetyltrimethylammoniumchlorid.

Überraschenderweise hat sich in eigenen Untersuchungen gezeigt, dass Hydroxyester der Formel (I) (und insbesondere diejenigen Hydroxyester, die vorstehend bereits als bevorzugt bezeichnet wurden) insbesondere in tensidhaltigen Zubereitungen, die das Tensid Natriumlaurylethersulfat umfassen, regelmäßig eine signifikante Erhöhung der Viskosität bewirken.

Ähnliche positive Ergebnisse wurden regelmäßig in eigenen Untersuchungen festgestellt für den Einsatz der Hydroxyester der Formel (I) in tensidhaltigen Zubereitungen, welche ein oder mehrere andere anionische Tenside umfassen, insbesondere andere anionische Tenside ausgewählt aus der Gruppe bestehend aus Sulfaten und Sulfonaten.

Es versteht sich, dass eine erfindungsgemäße O/W-Emulsion ebenso wie eine erfindungsgemäße (vorzugsweise einphasige) tensidhaltige Zubereitung immer neben dem Hydroxyester der Formel (I) oder den Hydroxyestern der Formel (I) (wie oben definiert, vorzugsweise wie oben als bevorzugt definiert) noch weitere Verbindungen umfasst.

Der Fachmann kann anhand weniger Vorversuche ermitteln, welche Gesamtmenge an dem Hydroxyester der Formel (I) oder den Hydroxyestern der Formel (I) er wählen muss, damit die erfindungsgemäße O/W-Emulsion beziehungsweise die erfindungsgemäße (vorzugsweise einphasige) tensidhaltige Zubereitung die erwünschten physikalischen Eigenschaften erhält. Er wird hierzu im Bedarfsfall unterschiedliche Gesamtmengen an Hydroxyestern der Formel (I) testen und ermitteln, (i) ab welcher Gesamtmenge und (ii) bis zu welcher Gesamtmenge die erwünschten Resultate erreicht werden. Hierzu wird der Fachmann Vergleichsuntersuchungen unter analogen Bedingungen durchführen.

Damit in einer erfindungsgemäßen (vorzugsweise einphasigen) tensidhaltigen Zubereitung der eine oder die mehreren Hydroxyester der Formel (I) (wie oben definiert, vorzugsweise wie oben als bevorzugt definiert) als Mittel zur Erhöhung der Viskosität (die vergleichsweise Erhöhung der Viskosität für erfindungsgemäße tensidhaltige Zubereitungen wurde bei 22,7 °C bestimmt) dienen können, werden sie in einer hierzu geeigneten Gesamtmenge eingesetzt. Hinsichtlich der Bestimmung einer insoweit geeigneten Gesamtmenge gelten die obigen Ausführungen zu einer für den Fachmann geeigneten Vorgehensweise entsprechend.

Die vorliegende Erfindung betrifft auch die Verwendung
(a) eines einzelnen Hydroxyesters der Formel (I) oder
(b) einer Mischung umfassend oder bestehend aus zwei oder mehr unterschiedlichen Hydroxyestern der Formel (I), 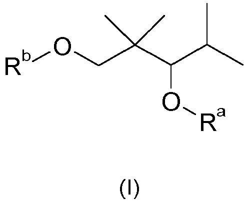 wobei jeweils einer der beiden Reste R^{a} oder R^{b} Wasserstoff und der jeweils andere Rest Ra oder R^{b} einen geradkettigen oder verzweigten Acylrest mit 2 bis 10 C-Atomen bedeutet,
   als Mittel zur Modifizierung einer oder mehrerer physikalischer Eigenschaften einer (beispielsweise kosmetischen oder medizinischen oder Reinigungs-)Formulierung, wobei die physikalischen Eigenschaften ausgewählt sind aus der Gruppe bestehend aus Emulsionsstabilität und Viskosität, vorzugsweise als Mittel zur Erhöhung der Emulsionsstabilität und/oder Viskosität einer solchen Formulierung.

Hinsichtlich bevorzugter Hydroxyester der Formel (I) gelten wiederum die vorstehenden Ausführungen zu bevorzugten erfindungsgemäßen Zubereitungen, erfindungsgemäßen O/W-Emulsionen und erfindungsgemäßen tensidhaltigen Zubereitungen entsprechend.

Bevorzugte Mittel zur Modifizierung der physikalischen Eigenschaft "Emulsionsstabilität" sind Emulgatoren; sie stabilisieren einer Emulsion.

Hinsichtlich des Verständnisses der Begriffe "Emulsionsstabilität" und "Viskosität" gelten die obigen Ausführungen wiederum entsprechend.

Es war überraschend und nicht vorhersehbar, dass die erfindungsgemäß einzusetzenden Hydroxyester der Formel (I) (wie oben definiert, vorzugsweise wie oben als bevorzugt bezeichnet) in der Lage sind, die Emulsionsstabilität von O/W-Emulsionen und die Viskosität von insbesondere tensidhaltigen Zubereitungen in der von der Industrie gewünschten Weise zu beeinflussen, d.h. üblicherweise zu erhöhen.

Das oben in Verbindung mit erfindungsgemäßen Zubereitungen Gesagte, insbesondere betreffend bevorzugte Acylreste, gilt entsprechend.

Bevorzugt ist insbesondere eine erfindungsgemäße Verwendung wie soeben definiert, wobei
- in der Alternative (a) der eine Hydroxyester bzw.
- in der Alternative (b) einer, zwei oder mehr der Hydroxyester

Hydroxyester der Formel (I) sind 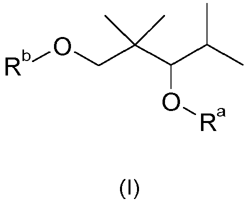 wobei jeweils einer der beiden Reste R^{a} oder R^{b} Wasserstoff und der jeweils andere Rest R^{a} oder R^{b} einen geradkettigen oder verzweigten Acylrest mit 2 bis 6 C-Atomen bedeutet.

Weiter bevorzugt ist es, wenn der Acylrest eines, zweier, mehrerer oder sämtlicher der Hydroxyester der Formel (I) ausgewählt ist aus der Gruppe bestehend aus Acetyl, Propionyl, n-Butyryl, Isobutyryl, Crotonyl, n-Pentanoyl, Isopentanoyl, n-Hexanoyl und Isohexanoyl,
vorzugsweise ausgewählt aus der Gruppe bestehend aus n-Butyryl, Isobutyryl und Crotonyl.

Weiter bevorzugt ist eine erfindungsgemäße Verwendung
- eines Hydroxyesters der Formel (I) wie oben definiert (vorzugsweise wie oben als bevorzugt bezeichnet), wobei der Rest R^{a} Wasserstoff und der Rest R^{b} einen geradkettigen oder verzweigten Acylrest bedeutet
   oder
- einer Mischung umfassend oder bestehend aus
   - einem ersten Hydroxyester der Formel (I) wie oben definiert (vorzugsweise wie oben als bevorzugt bezeichnet), wobei der Rest R^{a} Wasserstoff und der Rest R^{b} einen geradkettigen oder verzweigten Acylrest bedeutet sowie zusätzlich
   - einem zweiten Hydroxyester der Formel (I) wie oben definiert (vorzugsweise wie oben als bevorzugt bezeichnet).

Weiter bevorzugt ist eine erfindungsgemäße Verwendung (wie vorstehend definiert, vorzugsweise wie vorstehend als bevorzugt bezeichnet)
- einer Mischung umfassend oder bestehend aus
- einem ersten Hydroxyester der Formel (I) wie oben definiert (vorzugsweise wie oben als bevorzugt bezeichnet), wobei der Rest R^{a} Wasserstoff und der Rest R^{b} einen geradkettigen oder verzweigten Acylrest bedeutet
   und
- einem zweiten Hydroxyester der Formel (I) wie oben definiert (vorzugsweise wie oben als bevorzugt bezeichnet), wobei der Rest R^{b} Wasserstoff und der Rest R^{a} einen geradkettigen oder verzweigten Acylrest bedeutet,
wobei der Rest R^{a} des ersten Hydroxyesters die Bedeutung von R^{b} des zweiten Hydroxyesters hat und der Rest R^{b} des ersten Hydroxyesters die Bedeutung von R^{a} des zweiten Hydroxyesters hat.

Ganz besonders bevorzugt ist schließlich eine erfindungsgemäße Verwendung (wie vorstehend definiert, vorzugsweise wie vorstehend als bevorzugt bezeichnet)
eines Hydroxyesters der Formel (Ib) 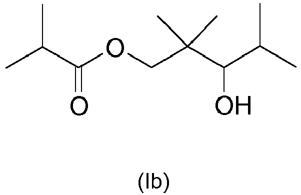 oder
einer Mischung umfassend oder bestehend aus einem ersten und einem zweiten Hydroxyester der Formel (I) wie oben definiert (vorzugsweise wie oben als bevorzugt bezeichnet), wobei der erste Hydroxyester der Formel (I) die Formel 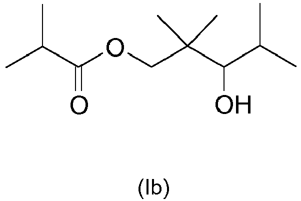 besitzt
und der zweite Hydroxyester der Formel (I) vorzugsweise die Formel 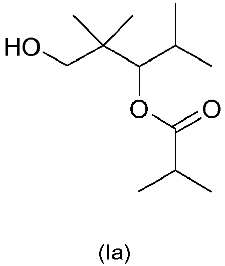 besitzt.

Die vorliegende Erfindung betrifft schließlich auch ein Verfahren
(a) zum Herstellen und/oder Stabilisieren einer O/W-Emulsion, mit folgenden Schritten:
   - Vermengen zweier Flüssigkeiten in Gegenwart einer emulgierend wirkenden Menge von einem, zwei oder mehr Hydroxyestern der Formel (I) wie oben definiert (vorzugsweise wie oben als bevorzugt bezeichnet).
   und/oder
(b) zum Herstellen einer tensidhaltigen Zubereitung mit erhöhter Viskosität, enthaltend ein oder mehrere anionische Tenside, mit folgenden Schritten:
   - Vermengen einer tensidhaltigen Flüssigkeit, enthaltend ein oder mehrere anionische Tenside, mit einer viskositätserhöhenden Menge von einem, zwei oder mehr Hydroxyestern der Formel (I) wie oben definiert (vorzugsweise wie oben als bevorzugt bezeichnet).

Bevorzugt ist somit gemäß einem ersten Aspekt der vorliegenden Erfindung ein Verfahren zum Herstellen und/oder Stabilisieren einer O/W-Emulsion, mit folgenden Schritten:
- Vermengen zweier Flüssigkeiten in Gegenwart einer emulgierend wirkenden Menge von einem, zwei oder mehr Hydroxyestern der Formel (I) wie vorstehend definiert (vorzugsweise wie oben als bevorzugt bezeichnet).

Hinsichtlich bevorzugter Hydroxyester der Formel (I) gelten selbstverständlich wiederum die weiter oben im Zusammenhang mit erfindungsgemäßen Zubereitungen gemachten Ausführungen.

Und bevorzugt ist somit gemäß einem zweiten Aspekt der vorliegenden Erfindung ein Verfahren zum Herstellen einer tensidhaltigen Zubereitung mit erhöhter Viskosität mit folgenden Schritten:
- Vermengen einer tensidhaltigen Flüssigkeit, enthaltend ein oder mehrere anionische Tenside, mit einer viskositätserhöhenden Menge von einem, zwei oder mehr Hydroxyestern der Formel (I) wie vorstehend definiert (vorzugsweise wie oben als bevorzugt bezeichnet).

Hinsichtlich der erfindungsgemäßen Verfahren gilt das oben Gesagte betreffend erfindungsgemäße Zubereitungen, erfindungsgemäße O/W-Emulsionen, erfindungsgemäße tensidhaltige Zubereitungen und erfindungsgemäße Verwendungen entsprechend. Nachfolgend werden bevorzugte Ausgestaltungen der vorliegenden Erfindung anhand von Beispielen näher erläutert.

### Beispiele:

Die nachfolgenden Beispiele zeigen beispielhafte Zusammensetzungen erfindungsgemäßer Zubereitungen und beschreiben ausgewählte Eigenschaften solcher Zubereitungen. Bei diesen Eigenschaften handelt es sich insbesondere um:
- die Partikelgrößenverteilung in O/W-Emulsionen
- die Stabilität einer Emulsion
- die Viskosität einer tensidhaltigen Zubereitung (Shampoo).

Sofern nicht anders angegeben, handelt es sich bei Mengenangaben in den Beispielen um Gewichtsprozentangaben (w/w %).

### 1. Partikelgrößenverteilung in O/W-Emulsionen

### 1.1 Probenherstellung

Die Bestimmung der Partikelgrößenverteilung wurde an sieben Proben mit den Probennummern 1 bis 7 (sieben O/W-Emulsionen) vorgenommen. Die Zusammensetzung jeder dieser sieben Proben ist in Tabelle 1 wiedergegeben. Die Formulierungsbestandteile wurden in zwei Gruppen (Phasen) A und B zusammengefasst. Die Abkürzung INCI bedeutet "Internationale Nomenklatur für kosmetische Inhaltsstoffe" (International Nomenclature of Cosmetic Ingredients) und bezieht sich auf die einzelnen Formulierungsbestandteile der sieben Proben.

**Tabelle 1**

| | | w/w % | | | | | | |
|---|---|---|---|---|---|---|---|---|
| | **Substanz (INCI)** | **1** | **2** | **3** | **4** | **5** | **6** | **7** |
| | Potassium Cetyl Phosphate, Hydrogenated Palm Glycerides | 2,0 | 2,0 | 2,0 | 2,0 | 2,0 | 2,0 | 2,0 |
| | Cetearyl Alcohol | 0,7 | 0,7 | 0,7 | 0,7 | 0,7 | 0,7 | 0,7 |
| | Caprylic/Capric Triglyceride | 8,0 | 8,0 | 8,0 | 8,0 | 8,0 | 8,0 | 8,0 |
| **A** | Cetearyl Ethylhexanoate | 4,0 | 4,0 | 4,0 | 4,0 | 4,0 | 4,0 | 4,0 |
| | Dimethicone | 0,1 | 0,1 | 0,1 | 0,1 | 0,1 | 0,1 | 0,1 |
| | 2-Methylpropanoic acid 3-hydroxy-2,2,4-trimethylpentyl ester | - | 0,5 | 1,0 | 2,0 | 3,0 | 4,0 | 5,0 |
| **B** | Water (Aqua) | 83,7 | 83,2 | 82,7 | 81,7 | 80,7 | 79,7 | 78,7 |
| | Glycerin | 1,5 | 1,5 | 1,5 | 1,5 | 1,5 | 1,5 | 1,5 |
| **TOTAL** | | 100 | 100 | 100 | 100 | 100 | 100 | 100 |

In einem ersten Arbeitsschritt wurden zur Herstellung einer jeden Probe die Formulierungsbestandteile der Phase A in den in Tabelle 1 angegebenen Mengenverhältnissen bereitgestellt, miteinander vermischt und auf eine Temperatur von ca. 80 °C erhitzt. In einem zweiten Arbeitsschritt wurden entsprechend die Formulierungsbestandteile der Phase B bereitgestellt, ebenfalls miteinander vermischt und ebenfalls auf ca. 80 °C erhitzt. In einem dritten Arbeitsschritt wurde die erhitzte Phase B zu der erhitzten Phase A gegeben, mit dieser vermischt und emulgiert (Ultra Turrax Rührer: 2 Minuten, 5000 U/min).

Die jeweilige Probe wurde anschließend mittels eines Blattrührers gerührt (150 U/min, 10 Minuten), bis die Probe auf annähernd Raumtemperatur abgekühlt war. Der pH-Wert jeder der Proben betrug 6,0. Es resultierten Proben mit den in Tabelle 1 angegebenen Probennummern 1 bis 7, wobei Probe 1 eine Referenzprobe war, die keine Verbindung der Formel (I) umfasste. Von jeder der hergestellten Proben 1 bis 7 wurde ein erster Anteil für die Bestimmung der Partikelgrößenverteilung genutzt. Ein zweiter Anteil der jeweils hergestellten Proben 1 bis 7 wurde für die Bestimmung der Stabilität genutzt (siehe weiter unten 2.).

Zum Erreichen einer guten Vergleichbarkeit der Proben wurde darauf geachtet, dass sämtliche Proben auf identische Weise hergestellt wurden. Aus Sicht der die Versuche durchführenden Fachleute unterschieden sich lediglich die chemischen Zusammensetzungen der Proben; die zur Herstellung durchgeführten Verfahrensschritte und eingestellten Verfahrensparameter (Temperatur, Rührbedingungen etc.) unterschieden sich nicht.

### 1.2 Bestimmung der Partikelgrößenverteilung

Zur Bestimmung der Partikelgrößenverteilung wurden in einem weiteren Arbeitsschritt die entsprechenden Anteile der Proben 1 bis 7 in einem Mastersizer Micro MAF 5000 (Hersteller: Malvern Instruments GmbH) untersucht. Hierzu wurden folgende Probenpräparationsschritte durchgeführt:

### 1.3 Ergebnisse der Bestimmung der Partikelgrößenverteilung

Das Ergebnis der Bestimmung der Partikelgrößenverteilung der Proben mit den Probennummern 1 bis 7 ist in Tabelle 2 wiedergegeben. Es wurden für jede der sieben Proben drei (volumenbezogene) Werte bestimmt, D(v, 0.1), D(v, 0.5) und D(v, 0.9), wobei
D(v, 0.1) bedeutet, dass 10 % der Partikel in der O/W-Emulsion einen Partikeldurchmesser aufweisen, der kleiner ist als der in der Tabelle angegebene Wert
D(v, 0.5) bedeutet, dass 50 % der Partikel in der O/W-Emulsion einen Partikeldurchmesser aufweisen, der kleiner ist als der in der Tabelle angegebene Wert
D(v, 0.9) bedeutet, dass 90 % der Partikel in der O/W-Emulsion einen Partikeldurchmesser aufweisen, der kleiner ist als der in der Tabelle angegebene Wert

**Tabelle 2**

| | **D(v, 0.1)** | **D(v, 0.5)** | **D(v, 0.9)** |
|---|---|---|---|
| **1** | 3,47 | 17,44 | 45,84 |
| **2** | 2,43 | 12,62 | 28,93 |
| **3** | 1,97 | 9,92 | 20,02 |
| **4** | 1,67 | 7,91 | 4,48 |
| **5** | 1,49 | 7,07 | 28,93 |
| **6** | 1,49 | 7,82 | 32,29 |
| **7** | 1,64 | 7,97 | 44,20 |

Für Probe 4 (umfassend 2 % eines Hydroxyesters der Formel (I)) wurde beispielsweise eine Partikelgrößenverteilung gemessen, für die gilt: D(v, 0.5) 7,91 und D(v, 0.9) 4,48. Probe 1 (umfassend keinen Hydroxyester der Formel 1) weist eine Partikelgrößenverteilung auf, für die gilt: D(v, 0.5) 17,44 und D(v, 0.9) 45,84. Im Vergleich mit Probe 1 weist Probe 4 somit eine Partikelgrößenverteilung auf, die eine große Menge kleinerer Partikel umfasst (insbesondere bezogen auf D(v, 0.5)).

Die Partikelgrößenverteilungskurven für D(v, 0.5) der Proben 1 und 4 sind graphisch in Figur 1 dargestellt. In Figur 1 bedeutet die durchgängig gezeichnete Kurve die Partikelgrößenverteilung in Probe 1, wohingegen die unterbrochen gezeichnete Kurve die Partikelgrößenverteilung in Probe 4 bedeutet. Auf der X-Achse sind die Partikeldurchmesser in µm aufgetragen. Die Partikelgrößenverteilungskurve der Partikel in Probe 4 ist deutlich zu kleineren Partikeln hin verschoben, verglichen mit der Partikelgrößenverteilungskurve der Partikel in Probe 1.

### 2. Stabilität einer O/W-Emulsion

### 2.1 Probenherstellung

Die Bestimmung der Stabilität von O/W-Emulsionen wurde an entsprechenden Anteilen der sieben Proben mit den Probennummern 1 bis 7 vorgenommen. Die Zusammensetzung dieser sieben Proben entspricht der Zusammensetzung wie in Tabelle 1 angegeben. Die Proben wurden wie unter 1.1 beschrieben hergestellt.

### 2.2 Bestimmung der Stabilität

Die Anteile der Proben 1 bis 7 wurden mittels eines LUMisizers der Firma LUM (LUM GmbH) untersucht. Der Lumisizer untersucht Entmischungsvorgänge (Entmischung von Öl- und wässriger Phase) von mehreren Proben gleichzeitig mittels Zentrifugation und der Messung von Transmissionsänderungen. Die Anteile der Proben werden in separate Küvetten eingefüllt und zentrifugiert; dabei wurde kontinuierlich das Profil der Transmission in Küvettenlängsrichtung bestimmt. Die Dauer der Analyse betrug 1,25 Stunden (dies entspricht einer Lagerung von in etwa einem Monat); die Analyse wurde bei einer Umdrehungsgeschwindigkeit von 2000 rpm und einer Temperatur von 25 °C durchgeführt. Die Breite des Küvettenbereiches, in dem die wässrige Phase vollständig entmischt vorliegt, nahm im Analysezeitraum beständig zu, da es durch die Zentrifugation zu einer fortschreitenden Entmischung kam. Dies ist im Transmissionsprofil unmittelbar zu erkennen, vgl. hierzu Fig. 3a und 3b (Details nachfolgend im Text).

### 2.3. Ergebnisse der LUMIsizer-Untersuchung und Bestimmung des Instabilitätsindexes

Es wurde festgestellt, dass sich die 7 Proben unter den Bedingungen gemäß 2.2 unterschiedlich stark entmischten. Sie besaßen somit eine unterschiedliche Emulsionsstabilität.

Der Grad der Entmischung jeder einzelnen Probe wurde vor, während und nach der Behandlung gemäß 2.2 quantitativ bestimmt (vergleiche hierzu Figur 3; die zeitliche Veränderung ist durch Überlagerung einzelner Profile dargestellt; eine Messung beinhaltet 255 Einzelprofile). In Figur 3 (X-Achse: Position in mm, Y-Achse: Transmission in %) haben die Bezeichnungen A, B, C, D, E und F die folgenden Bedeutungen:
A) Analyseküvette (analysing cuvette)
B) Meniskus (meniscus)
C) Grenzfläche (interface)
D) Zellenboden (cell bottom)
E) letztes Profil (last profile)
F) erstes Profil (first profile)

Zum Zeitpunkt t = 0 ist die gesamte Menge der Emulsion jeder einzelnen Probe in der Küvette homogen; das Transmissionsprofil zeigt, das im gesamten befüllten Bereich der Küvette die Transmission (aufgrund einer homogenen Verteilung von Ölpartikeln geringer Lichtdurchlässigkeit) gleichmäßig gering ist.

Mit zunehmender Zentrifugationsdauer bildet sich eine zunehmend große Menge einer vollständig entmischten wässrigen Phase aus; im Transmissionsprofil zeigt sich dies durch eine zunehmende Breite des Bereiches mit hoher Transmission im Transmissionsprofil (im Transmissionsprofil gemäß Fig. 3a bzw. 3b rechts, X-Achse: Position in mm, Y-Achse: Transmission in %) im Vergleich mit dem Bereich, in dem sich die Ölphase dispergiert oder separat befindet (im Transmissionsprofil gemäß Fig. 3a bzw. 3b links, X-Achse: Position in mm, Y-Achse: Transmission in %).

Die Breite der separaten wässrigen Phase in der Küvette nimmt mit zunehmender Zentrifugationsdauer zu. Die Breite des Bereiches geringer Lichtdurchlässigkeit nimmt in entsprechendem Maße ab. Dieser Umstand wird mittels des sogenannten Instabilitätsindexes zahlenmäßig ausgedrückt. Zu einem gegebenen Zeitpunkt t = 0 ist der Instabilitätsindex einer jeden Probe Null (kein Bereich der gefüllten Küvette besitzt eine hohe Transmission). Mit zunehmender Zeit nimmt der Wert für den Instabilitätsindex zu, so wie die Breite der wässrigen Phase in der Küvette und damit die Breite des Bereiches mit hoher Transmission im Transmissionsprofil zunimmt.

Der Instabilitätsindex (INDEX) der Proben 1 bis 7 wurde über die Zentrifugationsdauer (1,25 Stunden = 4500 Sekunden) hinweg durch den LUMIsizer bestimmt. Die Ergebnisse der Untersuchung sind in Figur 2 wiedergegeben, die Werte für den Zeitpunkt t = 4500s (= 1,25h) sind in Tabelle 3 angegeben Ein INDEX von 0 bedeutet sehr stabil, ein INDEX von 1 bedeutet vollständige Entmischung.

**Tabelle 3**

| | **INDEX** |
|---|---|
| 1 | 0,40 |
| 2 | 0,37 |
| 3 | 0,29 |
| 4 | 0,20 |
| 5 | 0,26 |
| 6 | 0,35 |
| 7 | 0,37 |

Nach einer Analysedauer von 1,25 Stunden erreicht Probe 4 (umfassend 2 Gew.-% eines Hydroxyesters der Formel (I)) einen Instabilitätsindex von 0,2 und damit den geringsten Wert unter den untersuchten Proben. Probe 4 besitzt somit über den Untersuchungszeitraum die geringste Instabilität (die geringste Entmischung von Öl- und wässriger Phase) und somit die höchste Stabilität in der Gruppe der Proben 1 bis 7.

Die über die gesamte Zentrifugationsdauer (1,25 Stunden = 4500 Sekunden) fast kontinuierlich bestimmten Instabilitätsindizes sind in Figur 2 graphisch (als Kurven) aufgetragen. Die Kurve der Probe 4 folgt einem linearen Verlauf, wohingegen die Kurve der Probe 1 bereits nach ca. 1000 bis 1500 Sekunden eine exponentielle Sättigung erfährt. Probe 4 ist somit nicht nur durch einen geringeren Instabilitätsindex (0,2) zum Zeitpunkt t = 4500s (= 1,25h) gegenüber Probe 1 (0,4) gekennzeichnet. Der Kurvenverlauf der Probe 4 zeigt, dass der Entmischungsvorgang in Probe 4 nicht nur zu einem geringeren Grad der Entmischung führt (zum Zeitpunkt t = 4500s (= 1,25h), sondern auch deutlich langsamer abläuft (linearer Kurvenverlauf), verglichen mit dem Entmischungsvorgang in Probe 1, welcher durch einen Kurvenverlauf mit exponentieller Sättigung beschrieben wird.

Die Figuren 3a und 3b zeigen die Transmissionsprofile zu Probe 1 (Figur 3a) und Probe 4 (Figur 3b).

Die Transmissionsprofile in den Figuren 3a und 3b sind das Ergebnis von jeweils 255 (übereinandergelegten) Einzelmessungen (Einzelprofile), die über die Zentrifugationsdauer von t = 4500s (= 1,25h) in gleichmäßigen Abständen aufgezeichnet wurden.

Das in Figur 3a dargestellte Transmissionsprofil der Probe 1 (umfassend keinen Hydroxyester der Formel (I)) zeigt die (durchstrahlte) wässrige Phase (ca. 70 % Transmission, rechts in der Figur 3a) im Bereich von ca. 120 bis ca. 130 mm. Der Bereich größer 130 mm entspricht bereits dem Boden der Küvette und somit dem Küvettenmaterial. Im Bereich von ca. 105 bis ca. 120 mm liegt die Ölphase der Probe 1 dispergiert oder separat vor (ca. 5 % Transmission, links in der Figur 3a). Der Bereich kleiner 105 mm entspricht dem gasgefüllten Raum außerhalb der Probe innerhalb der Küvette.

Das in Figur 3b dargestellte Transmissionsprofil der Probe 4 (umfassend 2 Gew.-% eines Hydroxyesters der Formel (I)) zeigt die (durchstrahlte) wässrige Phase (ca. 90 % Transmission, rechts in der Figur 3b) im Bereich von ca. 125 bis ca. 130 mm. Im Bereich von ca. 108 bis ca. 125 mm liegt die Ölphase der Probe 4 dispergiert oder separat vor (ca. 5 % Transmission, links in der Figur 3b).

Das Transmissionsprofil der Probe 4 (Figur 3b) zeigt somit eine weniger breite (durchstrahlte) wässrige Phase (im Bereich von ca. 125 bis ca. 130 mm) verglichen mit dem Transmissionsprofil der Probe 1 (Figur 3a, im Bereich von ca. 120 bis ca. 130 mm). Somit weist Probe 4 einen geringeren Grad der Entmischung als Probe 1 auf.

### 3. Viskosität einer tensidhaltigen Zubereitung (Shampoo)

### 3.1 Probenherstellung

Die Bestimmung der Viskosität wurde an vier Proben mit den Probennummern V1 bis V4 vorgenommen. Die Zusammensetzung jeder dieser vier Proben ist in Tabelle 4 wiedergegeben. Die Formulierungsbestandteile wurden in 4 Gruppen (Phasen) A bis D zusammengefasst. Die Abkürzung INCI bedeutet "Internationale Nomenklatur für kosmetische Inhaltsstoffe" (International Nomenclature of Cosmetic Ingredients) und bezieht sich auf die einzelnen Formulierungsbestandteile der vier Proben.

**Tabelle 4**

| | **Substanz INCI** | **V1** | **V2** | **V3** | **V4** |
|---|---|---|---|---|---|
| A | Sodium Laureth Sulfate, Lauryl Glycoside | 17,0 | 17,0 | 17,0 | 17,0 |
| | Citric Acid | 0,15 | 0,15 | 0,15 | 0,15 |
| | 1,2 Hexandiol, Caprylyl Glycol | 1,0 | 1,0 | 1,0 | 1,0 |
| | Disodium EDTA | 0,1 | 0,1 | 0,1 | 0,1 |
| | Sodium Chloride | 0,4 | 0,4 | 0,4 | 0,4 |
| B | Water (Auqa) | 76,0 | 75,5 | 75,0 | 74,0 |
| | Polyquaternium-10 | 0,2 | 0,2 | 0,2 | 0,2 |
| C | Potassium Sorbate | 0,15 | 0,15 | 0,15 | 0,15 |
| D | Cocoamidoproyl Betaine | 5,0 | 5,0 | 5,0 | 5,0 |
| | Trimethyl Hydroxypentyl Isobutyrate | - | 0,5 | 1,0 | 2,0 |
| | TOTAL | 100,0 | 100,0 | 100,0 | 100,0 |

In einem ersten Arbeitsschritt wurden die Formulierungsbestandteile der Phase A bereitgestellt und vorsichtig miteinander verrührt, bis eine erste Schaumbildung beobachtet wurde (der Rührvorgang wurde abgebrochen, sobald eine erste Schaumbildung beobachtet wurde). In einem zweiten Arbeitsschritt wurde das Polyquaternium-10 (Ucare Polymer JR-400) bereitgestellt, mit dem Wasser vermischt und die Mischung auf ca. 50 °C erhitzt, so dass das Polymer zu quellen begann. Der Quellvorgang wurde beendet, sobald die Dispersion klar wurde und eine leichte Viskosität beobachtet wurde. In einem dritten Arbeitsschritt wurden nacheinander die Formulierungsbestandteile der Phasen B, C und D unter kontinuierlichem Rühren der Phase A hinzugefügt. Anschließend wurde der pH der resultierenden Mischungen auf ca. 5,4 bis 5,8 eingestellt. Es resultierten die Proben V1 bis V4.

### 3.2 Bestimmung der Viskosität

Die Viskosität der Proben V1 bis V4 wurde bei einer Temperatur von 22,7 °C mittels eines Rotationsviskosimeters (HAAKE Viscotester 7 mit einer Spindel des Typs 3) bestimmt.

### 3.3. Ergebnisse der Viskositätsbestimmung

Das Ergebnis der Viskositätsbestimmung der Proben mit den Probennummern V1 bis V4 ist in Tabelle 5 wiedergegeben.

**Tabelle 5**

| | **V1** | **V2** | **V3** | **V4** |
|---|---|---|---|---|
| [mPas] | 480 | 1240 | 1324 | 566 |

Probe V3 (umfassend 1 Gew.-% eines Hydroxyesters der Formel (I)) wies die vergleichsweise höchste Viskosität auf (1324 mPas). Probe V1 (umfassend keinen Hydroxyester der Formel (I)) wies eine Viskosität von 480 mPas auf und besaß somit eine vergleichsweise deutlich reduzierte Viskosität verglichen mit Probe V3.

### 4. Weitere Formulierungsbeispiele

### 4.1 Duschbad/Shampoo

| **Ausgangsmaterial** | **Substanz INCI** | **w/w %** |
|---|---|---|
| Water, demin. | Water (Aqua) | 76,7 |
| Plantacare PS 10 | Sodium Laureth Sulfate, Lauryl Glucoside | 20,0 |
| SymDiol 68, 108580 | Hexylene Glycol, Caprylyl Glycol | 1,0 |
| EDTA BD | Disodium EDTA | 0,1 |
| Sodium Chloride | Sodium Chloride | 1,0 |
| Citric Acid, cryst. | Citric Acid | 0,2 |
| Hydroxyester d. Formel (I) | Trimethyl Hydroxypentyl Isobutyrate | 1,0 |

### 4.2 Standard O/W-Emulsion

| **Ausgangsmaterial** | **Substanz INCI** | **w/w %** |
|---|---|---|
| Emulsiphos | Potassium Cetyl Phosphate, Hydrogenated Palm Glycerides | 2,0 |
| Cutina PES | Pentaerythrityl Distearate | 2,0 |
| PCL Solid | Stearyl Heptanoate, Stearyl Caprylate | 2,0 |
| Lanette O | Cetearyl Alcohol | 2,5 |
| PCL Liquid 100 | Cetearyl Ethylhexanoate | 5,0 |
| Isodragol | Triisononanoin | 2,0 |
| Dow Corning 246 Fluid | Cyclohexasiloxane, Cyclopentasiloxane | 2,0 |
| Dragoxat 89 | Ethylhexyl Isononanoate | 3,0 |
| Ultrez-10 | Carbomer | 0,2 |
| Wasser | Water (Aqua) | 74,1 |
| Hydroxyester d. Formel (I) | Trimethyl Hydroxypentyl Isobutyrate | 5,0 |
| Sodium Hydroxide 10% sol. | Sodium Hydroxide | 0,2 |
| TOTAL | | 100 |
| pH | | 6,0 |

## Patentansprüche

1. Zubereitung umfassend
- einen oder mehrere Hydroxyester der Formel (I) 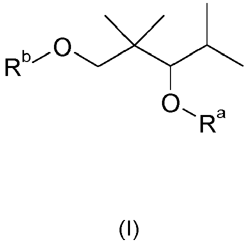 wobei jeweils einer der beiden Reste R^{a} oder R^{b} Wasserstoff und der jeweils andere Rest R^{a} oder R^{b} einen geradkettigen oder verzweigten Acylrest mit 2 bis 10 C-Atomen bedeutet,
sowie
- ein oder mehrere weitere Verbindungen,
wobei die Zubereitung eine O/W-Emulsion ist, für deren Partikelgrößenverteilung gilt: D(v, 0.9) = 40 µm oder weniger, wobei in der Zubereitung die Gesamtmenge an dem oder den Hydroxyestern der Formel (I) so gewählt ist, dass die O/W-Emulsion im Vergleich mit einer Vergleichsemulsion, die bei ansonsten gleicher Zusammensetzung keinen Hydroxyester der Formel (I) umfasst, eine erhöhte Emulsionsstabilität besitzt
und/oder
wobei die Zubereitung eine tensidhaltige Zubereitung ist, enthaltend ein oder mehrere anionische Tenside, und die Gesamtmenge an dem oder den Hydroxyestern der Formel (I) in der Zubereitung so gewählt ist, dass die Zubereitung im Vergleich mit einer Vergleichszubereitung, die bei ansonsten gleicher Zusammensetzung keinen Hydroxyester der Formel (I) umfasst, bei 22,7 °C eine erhöhte Viskosität besitzt.

2. Zubereitung nach Anspruch 1, umfassend
- einen oder mehrere Hydroxyester der Formel (I) 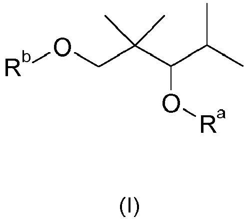 wobei jeweils einer der beiden Reste R^{a} oder R^{b} Wasserstoff und der jeweils andere Rest R^{a} oder R^{b} einen geradkettigen oder verzweigten Acylrest mit 2 bis 6 C-Atomen bedeutet,
wobei die Zubereitung vorzugsweise eine solche O/W-Emulsion ist, für deren Partikelgrößenverteilung gilt: D(v, 0.9) = 40 µm oder weniger, und bei der die Gesamtmenge an diesem oder diesen Hydroxyestern der Formel (I) mit einem Acylrest mit 2 bis 6 C-Atomen in der Zubereitung so gewählt ist, dass die O/W-Emulsion im Vergleich mit einer Vergleichsemulsion, die bei ansonsten gleicher Zusammensetzung keinen solchen Hydroxyester der Formel (I) mit einem Acylrest mit 2 bis 6 C-Atomen umfasst, eine erhöhte Emulsionsstabilität besitzt
und/oder
wobei die Zubereitung vorzugsweise eine solche tensidhaltige Zubereitung ist, bei der die Gesamtmenge an diesem oder diesen Hydroxyestern der Formel (I) mit einem Acylrest mit 2 bis 6 C-Atomen in der Zubereitung so gewählt ist, dass die Zubereitung im Vergleich mit einer Vergleichszubereitung, die bei ansonsten gleicher Zusammensetzung keinen solchen Hydroxyester der Formel (I) mit einem Acylrest mit 2 bis 6 C-Atomen umfasst, bei 22,7 °C eine erhöhte Viskosität besitzt.

3. Zubereitung nach einem der vorangehenden Ansprüche, wobei der Acylrest eines, zweier, mehrerer oder sämtlicher der Hydroxyester der Formel (I) ausgewählt ist aus der Gruppe bestehend aus Acetyl, Propionyl, n-Butyryl, Isobutyryl, Crotonyl, n-Pentanoyl, Isopentanoyl, n-Hexanoyl und Isohexanoyl,
vorzugsweise ausgewählt aus der Gruppe bestehend aus n-Butyryl, Isobutyryl und Crotonyl.

4. Zubereitung nach einem der vorangehenden Ansprüche, umfassend
- einen ersten Hydroxyester der Formel (I) wie in Anspruch 1, 2 oder 3 definiert, wobei der Rest R^{a} Wasserstoff und der Rest R^{b} einen geradkettigen oder verzweigten Acylrest bedeutet
sowie gegebenenfalls zusätzlich
- einen zweiten Hydroxyester der Formel (I) wie in Anspruch 1, 2 oder 3 definiert, wobei der Rest R^{b} Wasserstoff und der Rest R^{a} einen geradkettigen oder verzweigten Acylrest bedeutet, wobei dieser Acylrest R^{a} des zweiten Hydroxyesters die gleiche Struktur wie der Acylrest R^{b} des ersten Hydroxyester hat,
wobei die Zubereitung vorzugsweise eine O/W-Emulsion ist, für deren Partikelgrößenverteilung gilt: D(v, 0.9) = 40 µm oder weniger, wobei die Gesamtmenge an dem ersten und dem zweiten Hydroxyester der Formel (I) in der Zubereitung vorzugsweise so gewählt ist, dass die O/W-Emulsion im Vergleich mit einer Vergleichsemulsion, die bei ansonsten gleicher Zusammensetzung keinen solchen ersten und zweiten Hydroxyester der Formel (I) umfasst, eine erhöhte Emulsionsstabilität besitzt
und/oder
wobei die Gesamtmenge an dem ersten und dem zweiten Hydroxyester der Formel (I) in der Zubereitung vorzugsweise so gewählt ist, dass die Zubereitung im Vergleich mit einer Vergleichszubereitung, die bei ansonsten gleicher Zusammensetzung keinen solchen ersten und zweiten Hydroxyester der Formel (I) umfasst, bei 22,7 °C eine erhöhte Viskosität besitzt.

5. Zubereitung nach Anspruch 4, wobei der erste Hydroxyester der Formel (I) die Formel 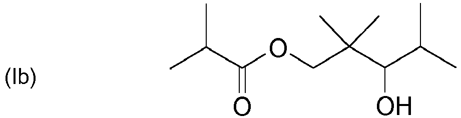 besitzt
und der gegebenenfalls vorhandene zweite Hydroxyester der Formel (I) die Formel 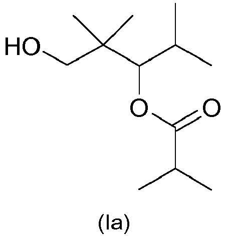 besitzt.

6. O/W-Emulsion, vorzugsweise nach einem der Ansprüche 1 bis 5, umfassend als Emulgator einen oder mehrere Hydroxyester der Formel (I) wie in einem der Ansprüche 1 bis 5 definiert, mit einer Partikelgrößenverteilung, für die gilt:
D(v, 0.9) = 40 µm oder weniger, vorzugsweise D(v, 0.9) = 30 µm oder weniger,
D (v, 0.5) = 20 µm oder weniger, vorzugsweise D (v, 0.5) = 15 µm oder weniger
und vorzugsweise zusätzlich:
D (v, 0.1) = 2,5 µm oder weniger, vorzugsweise D (v, 0.1) = 2,0 µm oder weniger.

7. O/W-Emulsion, vorzugsweise nach einem der Ansprüche 1 bis 6, umfassend als Emulgator einen oder mehrere Hydroxyester der Formel (I) wie in einem der Ansprüche 1 bis 5 definiert,
wobei die Stabilität der Emulsion so eingestellt ist, dass nach einer Lagerung der Zubereitung von 3 Wochen (21 Tage) bei 25 °C und einem Druck von 1013 HPa unter Luftabschluss für die Partikelgrößenverteilung gilt: D(v, 0.9) (t_{21d}) : D(v, 0.9) (t_{0d}) < 2, bevorzugt < 1,5, besonders bevorzugt < 1,2,
oder
wobei der Instabilitätsindex der O/W-Emulsion 75 % oder weniger, vorzugsweise 65 % oder weniger, besonders vorzugsweise 55 % oder weniger beträgt, verglichen mit dem Instabilitätsindex einer Vergleichs-O/W-Emulsion, die bei ansonsten gleicher Zusammensetzung keinen Hydroxyester der Formel (I) umfasst.

8. Tensidhaltige Zubereitung, vorzugsweise nach einem der Ansprüche 1 bis 5, umfassend
- als Mittel zur Erhöhung der Viskosität bei 22,7 °C einen oder mehrere Hydroxyester der Formel (I) wie in einem der Ansprüche 1 bis 5 definiert,
- ein oder mehrere anionische Tenside, vorzugsweise ausgewählt aus der Gruppe bestehend aus Sulfaten und Sulfonaten, besonders bevorzugt umfassend Natriumlaurylethersulfat
sowie gegebenenfalls
- ein oder mehrere weitere Tenside.

9. Verwendung
(a) eines einzelnen Hydroxyesters der Formel (I) oder
(b) einer Mischung umfassend oder bestehend aus zwei oder mehr unterschiedlichen Hydroxyestern der Formel (I), 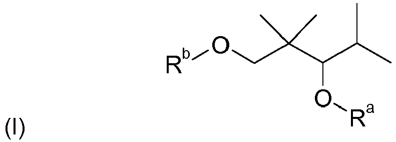 wobei jeweils einer der beiden Reste R^{a} oder R^{b} Wasserstoff und der jeweils andere Rest R^{a} oder R^{b} einen geradkettigen oder verzweigten Acylrest mit 2 bis 10 C-Atomen bedeutet,
als Mittel zur Modifizierung einer oder mehrerer physikalischer Eigenschaften einer Formulierung, wobei die physikalischen Eigenschaften ausgewählt sind aus der Gruppe bestehend aus Emulsionsstabilität und Viskosität, vorzugsweise als Mittel zur Erhöhung der Emulsionsstabilität und/oder Viskosität einer solchen Formulierung.

10. Verwendung nach Anspruch 9,
wobei
- in der Alternative (a) der eine Hydroxyester bzw.
- in der Alternative (b) einer, zwei oder mehr der Hydroxyester
Hydroxyester der Formel (I) sind 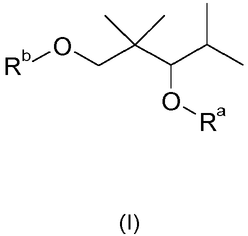 wobei jeweils einer der beiden Reste R^{a} oder R^{b} Wasserstoff und der jeweils andere Rest R^{a} oder R^{b} einen geradkettigen oder verzweigten Acylrest mit 2 bis 6 C-Atomen bedeutet.

11. Verwendung nach Anspruch 10, wobei der Acylrest eines, zweier, mehrerer oder sämtlicher der Hydroxyester der Formel (I) ausgewählt ist aus der Gruppe bestehend aus Acetyl, Propionyl, n-Butyryl, Isobutyryl, Crotonyl, n-Pentanoyl, Isopentanoyl, n-Hexanoyl und Isohexanoyl,
vorzugsweise ausgewählt aus der Gruppe bestehend aus n-Butyryl, Isobutyryl und Crotonyl.

12. Verwendung nach einem der vorangehenden Ansprüche 9 bis 11,
- eines Hydroxyesters der Formel (I) wie in Anspruch 1, 2 oder 3 definiert, wobei der Rest R^{a} Wasserstoff und der Rest R^{b} einen geradkettigen oder verzweigten Acylrest bedeutet
oder
- einer Mischung umfassend oder bestehend aus
- einem ersten Hydroxyester der Formel (I) wie in Anspruch 1, 2 oder 3 definiert, wobei der Rest R^{a} Wasserstoff und der Rest R^{b} einen geradkettigen oder verzweigten Acylrest bedeutet
sowie zusätzlich
- einem zweiten Hydroxyester der Formel (I) wie in Anspruch 1, 2 oder 3 definiert.

13. Verwendung nach einem der vorangehenden Ansprüche 9 bis 12,
- einer Mischung umfassend oder bestehend aus
- einem ersten Hydroxyester der Formel (I) wie in Anspruch 1, 2 oder 3 definiert, wobei der Rest R^{a} Wasserstoff und der Rest R^{b} einen geradkettigen oder verzweigten Acylrest bedeutet
und
- einem zweiten Hydroxyester der Formel (I) wie in Anspruch 1, 2 oder 3 definiert, wobei der Rest R^{b} Wasserstoff und der Rest R^{a} einen geradkettigen oder verzweigten Acylrest bedeutet,
wobei der Rest R^{a} des ersten Hydroxyesters die Bedeutung von R^{b} des zweiten Hydroxyesters hat und der Rest R^{b} des ersten Hydroxyesters die Bedeutung von R^{a} des zweiten Hydroxyesters hat.

14. Verwendung nach Anspruch 12 oder 13
eines Hydroxyesters der Formel (Ib) 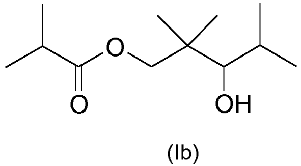 oder
einer Mischung umfassend oder bestehend aus einem ersten und einem zweiten Hydroxyester der Formel (I) wie in Anspruch 1, 2 oder 3 definiert,
wobei der erste Hydroxyester der Formel (I) die Formel 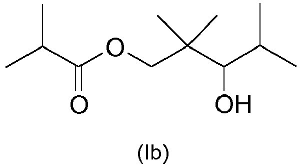 besitzt
und der zweite Hydroxyester der Formel (I) vorzugsweise die Formel 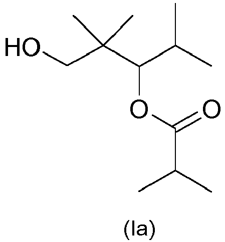 besitzt.

15. Verfahren
(a) zum Herstellen und/oder Stabilisieren einer O/W-Emulsion, mit folgenden Schritten:
- Vermengen zweier Flüssigkeiten in Gegenwart einer emulgierend wirkenden Menge von einem, zwei oder mehr Hydroxyestern der Formel (I) wie in einem der Ansprüche 1 bis 5 definiert.
und/oder
(b) zum Herstellen einer tensidhaltigen Zubereitung mit erhöhter Viskosität, enthaltend ein oder mehrere anionische Tenside, mit folgenden Schritten:
- Vermengen einer tensidhaltigen Flüssigkeit, enthaltend ein oder mehrere anionische Tenside, mit einer viskositätserhöhenden Menge von einem, zwei oder mehr Hydroxyestern der Formel (I) wie in einem der Ansprüche 1 bis 5 definiert.

## Claims

1. Preparation comprising
- one or more hydroxyester(s) of the formula (I), 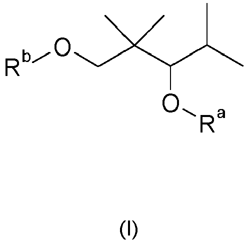 wherein in each case one of the two moieties R^{a} or R^{b} represents hydrogen and the other moiety R^{a} or R^{b} in each case represents a straight-chain or branched acyl moiety having 2 to 10 carbon atoms,
and
- one or more further compounds,
wherein the preparation is an O/W emulsion, for which particle size distribution the following applies: D(v, 0.9) = 40 µm or less, wherein in the preparation the total quantity of the hydroxyester(s) of the formula (I) is selected in such a way that the O/W emulsion, compared to a comparison emulsion which having an otherwise identical composition does not comprise any hydroxyester of the formula (I), has an increased emulsion stability,
and/or
wherein the preparation is a surfactant-containing preparation comprising one or more anionic surfactants and the total quantity of the hydroxyester(s) of the formula (I) in the preparation is selected in such a way that the preparation, compared to a comparison preparation which having an otherwise identical composition does not comprise a hydroxyester of the formula (I), has an increased viscosity at 22.7 °C.

2. Preparation according to claim 1, comprising
- one or more hydroxyester(s) of the formula (I) 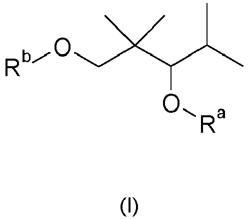 wherein in each case one of the two moieties R^{a} or R^{b} represents hydrogen and the other moiety R^{a} or R^{b} in each case represents a straight-chain or branched acyl moiety having 2 to 6 carbon atoms,
wherein the preparation is preferably such an O/W emulsion, for which particle size distribution the following applies: D(v, 0.9) = 40 µm or less, and in which the total quantity of these hydroxyester(s) of the formula (I) having an acyl moiety having 2 to 6 carbon atoms in the preparation is selected in such a way that the O/W emulsion, compared to a comparison emulsion which having an otherwise identical composition does not comprise any such hydroxyester(s) of the formula (I) having an acyl moiety having 2 to 6 carbon atoms, has an increased emulsion stability,
and/or
wherein the preparation is preferably such a surfactant-containing preparation, in which the total quantity of these hydroxyesters of the formula (I) having an acyl moiety having 2 to 6 carbon atoms in the preparation is selected in such a way that the preparation, compared to a comparison preparation which having an otherwise identical composition does not comprise any such hydroxyester of the formula (I) having an acyl moiety having 2 to 6 carbon atoms, has an increased viscosity at 22.7 °C.

3. Preparation according to any one of the preceding claims, wherein the acyl moiety of one, two, several or all of the hydroxyester(s) of the formula (I) is selected from the group consisting of acetyl, propionyl, n-butyryl, isobutyryl, crotonyl, n-pentanoyl, isopentanoyl, n-hexanoyl and isohexanoyl,
preferably selected from the group consisting of n-butyryl, isobutyryl and crotonyl.

4. Preparation according to any one of the preceding claims, comprising
- a first hydroxyester of the formula (I) as defined in claim 1, 2 or 3, wherein the moiety R^{a} represents hydrogen and the moiety R^{b} represents a straight-chain or branched acyl moiety
and optionally additionally
- a second hydroxyester of the formula (I) as defined in claim 1, 2 or 3, wherein the moiety R^{b} represents hydrogen and the moiety R^{a} represents a straight-chain or branched acyl moiety, wherein this acyl moiety R^{a} of the second hydroxyester has the same structure as the acyl moiety R^{b} of the first hydroxyester,
wherein the preparation is preferably an O/W emulsion, the particle size distribution for which the following applies: D(v, 0.9) = 40 µm or less, wherein the total quantity of the first hydoxyester and the second hydroxyester of the formula (I) in the preparation is preferably selected in such a way that the O/W emulsion, compared to a comparison emulsion which having an otherwise identical composition does not comprise any such first hydroxyester or second hydroxyester of the formula (I), has an increased emulsion stability,
and/or
wherein the total quantity of the first hydroxyester and the second hydroxyester of the formula (I) in the preparation is preferably selected in such a way that the preparation, compared to a comparison preparation which having an otherwise identical composition does not comprise any such first hydroxyester or second hydroxyester of the formula (I), has an increased viscosity at 22.7 °C.

5. Preparation according to claim 4, wherein the first hydroxyester of the formula (I) has the formula 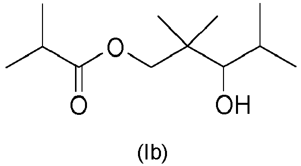 and the optionally present second hydroxyester of the formula (I) has the formula

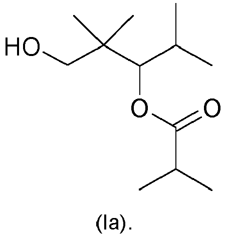

6. O/W emulsion, preferably according to any one of claims 1 to 5, comprising as the emulsifying agent one or more hydroxyester(s) of the formula (I) as defined in any one of claims 1 to 5, having a particle size distribution for which the following applies:
D(v, 0.9) = 40 µm or less, preferably D(v, 0.9) = 30 µm or less,
D (v, 0.5) = 20 µm or less, preferably D (v, 0.5) = 15 µm or less
and preferably additionally:
D (v, 0.1) = 2.5 µm or less, preferably D (v, 0.1) = 2.0 µm or less.

7. O/W emulsion, preferably according to any one of claims 1 to 6, comprising as the emulsifying agent one or more hydroxyester(s) of the formula (I) as defined in any one of claims 1 to 5,
wherein the stability of the emulsion is set in such a way that after storing the preparation for 3 weeks (21 days) at 25°C and at a pressure of 1013 HPa hermetically sealed, the following applies for the particle size distribution: D(v, 0.9) (t_{21d}) : D(v, 0.9) (t_{0d}) < 2, preferably < 1.5, particularly preferably < 1.2,
or
wherein the instability index of the O/W emulsion is 75% or less, preferably 65% or less, particularly preferably 55% or less, compared to the instability index of a comparison O/W emulsion which otherwise having an identical composition does not comprise any hydroxyester of the formula (I).

8. Surfactant-containing preparation, preferably according to any one of claims 1 to 5, comprising
- one or more hydroxyester(s) of the formula (I) as defined in any one of claims 1 to 5 as agent for increasing the viscosity at 22.7°C,
- one or more anionic surfactants, preferably selected from the group consisting of sulphates and sulphonates, particularly preferably comprising sodium lauryl ether sulphate
and optionally
- one or more further surfactants.

9. Use
(a) of a single hydroxyester of the formula (I) or
(b) of a mixture comprising or consisting of two or more different hydroxyester(s) of the formula (I), 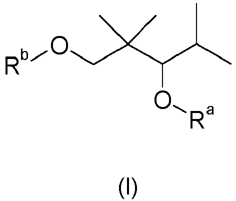 wherein in each case one of the two moieties R^{a} or R^{b} represents hydrogen and the other moiety R^{a} or R^{b} in each case represents a straight-chain or branched acyl moiety having 2 to 10 carbon atoms,
as agent for modifying one or more physical properties of a formulation, wherein the physical properties are selected from the group consisting of emulsion stability and viscosity, preferably as the means for increasing the emulsion stability and/or viscosity of such a formulation.

10. Use according to claim 9,
wherein
- in the alternative (a) the one hydroxyester or
- in the alternative (b) one, two or more of the hydroxyester(s)
are hydroxyesters of the formula (I) 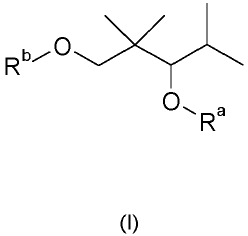 wherein in each case one of the two moieties R^{a} or R^{b} represents hydrogen and the other moiety R^{a} or R^{b} in each case represents a straight-chain or branched acyl moiety having 2 to 6 carbon atoms.

11. Use according to claim 10, wherein the acyl moiety of one, two, several or all of the hydroxyester(s) of the formula (I) is selected from the group consisting of acetyl, propionyl, n-butyryl, isobutyryl, crotonyl, n-pentanoyl, isopentanoyl, n-hexanoyl and isohexanoyl, preferably selected from the group consisting of n-butyryl, isobutyryl and crotonyl.

12. Use according to any one of the preceding claims 9 to 11,
- of a hydroxyester of the formula (I) as defined in claim 1, 2 or 3, wherein the moiety R^{a} represents hydrogen and the moiety R^{b} represents a straight-chain or branched acyl moiety
or
- of a mixture comprising or consisting of
- a first hydroxyester of the formula (I) as defined in claim 1, 2 or 3, wherein the moiety R^{a} represents hydrogen and the moiety R^{b} represents a straight-chain or branched acyl moiety
and additionally
- a second hydroxyester of the formula (I) as defined in claim 1, 2 or 3.

13. Use according to any one of the preceding claims 9 to 12,
- of a mixture comprising or consisting of
- a first hydroxyester of the formula (I) as defined in claim 1, 2 or 3, wherein the moiety R^{a} represents hydrogen and the moiety R^{b} represents a straight-chain or branched acyl moiety
and
- a second hydroxyester of the formula (I) as defined in claim 1, 2 or 3, wherein the moiety R^{b} represents hydrogen and the moiety R^{a} represents a straight-chain or branched acyl moiety,
wherein the moiety R^{a} of the first hydroxyester has the meaning of R^{b} of the second hydroxyester and the moiety R^{b} of the first hydroxyester has the meaning of R^{a} of the second hydroxyester.

14. Use according to claim 12 or 13
of a hydroxyester of the formula (lb) 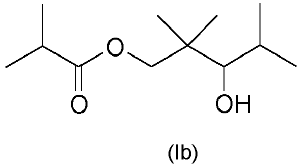 or
of a mixture comprising or consisting of a first hydroxyester and a second hydroxyster of the formula (I) as defined in claim 1, 2 or 3,
wherein the first hydroxyester of the formula (I) has the formula 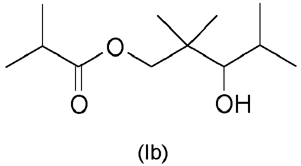 and the second hydroxyester of the formula (I) preferably has the formula

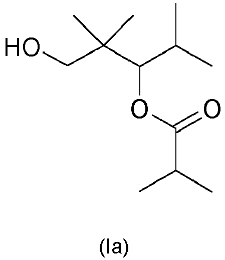

15. Method
(a) for manufacturing and/or stabilising an O/W emulsion, having the following steps:
- mixing two liquids in the presence of a quantity of one, two or more hydroxyester(s) of the formula (I) acting in an emulsifying manner as defined in any one of claims 1 to 5
and/or
(b) for producing a surfactant-containing preparation with increased viscosity having the following steps:
- mixing a surfactant-containing liquid with a quantity of one, two or more hydroxyester(s) of the formula (I) which increases the viscosity as defined in any one of claims 1 to 5.

## Revendications

1. Préparation comprenant
- un ou plusieurs hydroxyesters de formule (I) 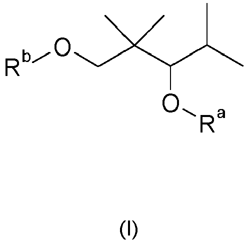 dans laquelle respectivement l'un des deux radicaux R^{a} ou R^{b} est l'hydrogène et l'autre radical R^{a} ou R^{b} respectif signifie un radical acyle à chaîne droite ou ramifié ayant de 2 à 10 atomes de carbone,
ainsi que
- un ou plusieurs autres composés,
la préparation étant une émulsion H/E pour la distribution granulométrique de laquelle s'applique: D(v, 0.9) = 40 µm ou moins, la quantité totale du ou des hydroxyester(s) de formule (I) dans la préparation étant choisie de telle sorte que l'émulsion H/E présente une stabilité d'émulsion accrue par rapport à une émulsion de comparaison qui, avec une composition par ailleurs identique, ne comprend pas d'hydroxyester de formule (I)
et/ou
la préparation étant une préparation contenant un tensioactif, qui contient un ou plusieurs tensioactif(s) anionique(s), et la quantité totale du ou des hydroxyester(s) de formule (I) dans la préparation étant choisie de telle sorte que la préparation présente une viscosité accrue à 22,7 °C par rapport à une préparation de comparaison qui, avec une composition par ailleurs identique, ne comprend pas d'hydroxyester de formule (I).

2. Préparation selon la revendication 1, comprenant
- un ou plusieurs hydroxyesters de formule (I) 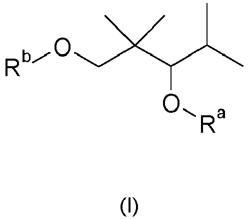 dans laquelle respectivement l'un des deux radicaux R^{a} ou R^{b} est l'hydrogène et l'autre radical R^{a} ou R^{b} respectif signifie un radical acyle à chaîne droite ou ramifié ayant de 2 à 6 atomes de carbone,
la préparation étant de préférence une telle émulsion H/E pour la distribution granulométrique de laquelle s'applique: D(v, 0.9) = 40 µm ou moins, et dans laquelle la quantité totale de ce ou ces hydroxyester(s) de formule (I) avec un radical acyle ayant de 2 à 6 atomes de carbone dans la préparation est choisie de telle sorte que l'émulsion H/E présente une stabilité d'émulsion accrue par rapport à une émulsion de comparaison qui, avec une composition par ailleurs identique, ne comprend pas un tel hydroxyester de formule (I) avec un radical acyle ayant de 2 à 6 atomes de carbone
et/ou
la préparation étant de préférence une telle préparation contenant un tensioactif dans laquelle la quantité totale de ce ou ces hydroxyester(s) de formule (I) avec un radical acyle ayant de 2 à 6 atomes de carbone dans la préparation est choisie de telle sorte que la préparation présente une viscosité accrue à 22,7 °C par rapport à une préparation de comparaison qui, avec une composition par ailleurs identique, ne comprend pas un tel hydroxyester de formule (I) avec un radical acyle ayant de 2 à 6 atomes de carbone.

3. Préparation selon l'une quelconque des revendications précédentes, dans laquelle le radical acyle d'un, de deux, de plusieurs ou de tous les hydroxyesters de formule (I) est choisi dans le groupe constitué par l'acétyle, le propionyle, le n-butyryle, l'isobutyryle, le crotonyle, le n-pentanoyle, l'isopentanoyle, le n-hexanoyle et l'isohexanoyle,
de préférence choisi dans le groupe constitué par le n-butyryle, l'isobutyryle et le crotonyle.

4. Préparation selon l'une quelconque des revendications précédentes, comprenant
- un premier hydroxyester de formule (I) tel que défini dans la revendication 1, 2 ou 3, dans lequel le radical R^{a} est l'hydrogène et le radical R^{b} signifie un radical acyle à chaîne droite ou ramifié
ainsi que, le cas échéant, en sus
- un deuxième hydroxyester de formule (I) tel que défini dans la revendication 1, 2 ou 3, dans lequel le radical R^{b} est l'hydrogène et le radical R^{a} est un radical acyle à chaîne droite ou ramifié, ce radical acyle R^{a} du deuxième hydroxyester présentant la même structure que le radical acyle R^{b} du premier hydroxyester,
la préparation étant de préférence une émulsion H/E pour la distribution granulométrique de laquelle s'applique: D(v, 0.9) = 40 µm ou moins, la quantité totale des premier et deuxième hydroxyesters de formule (I) dans la préparation étant choisie de préférence de telle sorte que l'émulsion H/E présente une stabilité d'émulsion accrue par rapport à une émulsion de comparaison qui, avec une composition par ailleurs identique, ne comprend pas de tels premier et deuxième hydroxyesters de formule (I)
et/ou
la quantité totale des premier et deuxième hydroxyesters de formule (I) dans la préparation étant de préférence choisie de telle sorte que la préparation présente une viscosité accrue à 22,7 °C par rapport à une préparation de comparaison qui, avec une composition par ailleurs identique, ne comprend pas de tels premier et deuxième hydroxyesters de formule (I).

5. Préparation selon la revendication 4, dans laquelle le premier hydroxyester de formule (I) présente la formule 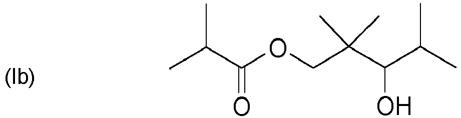 et le deuxième hydroxyester de formule (I), le cas échéant existant, présente la formule

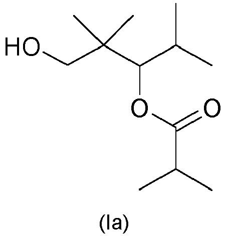

6. Emulsion H/E, de préférence selon l'une quelconque des revendications 1 à 5, comprenant comme émulsifiant un ou plusieurs hydroxyester(s) de formule (I) tel(s) que défini(s) dans l'une quelconque des revendications 1 à 5, avec une distribution granulométrique pour laquelle s'applique:
D(v, 0.9) = 40 µm ou moins, de préférence D(v, 0.9) = 30 µm ou moins,
D(v, 0.5) = 20 µm ou moins, de préférence D(v, 0.5) = 15 µm ou moins,
et, de préférence, en sus:
D(v, 0.1) = 2,5 µm ou moins, de préférence D(v, 0.1) = 2,0 µm ou moins.

7. Emulsion H/E, de préférence selon l'une quelconque des revendications 1 à 6, comprenant comme émulsifiant un ou plusieurs hydroxyester(s) de formule (I) tel(s) que défini(s) dans l'une quelconque des revendications 1 à 5,
la stabilité de l'émulsion étant réglée de telle sorte que, après un stockage de la préparation pendant 3 semaines (21 jours) à 25 °C et à une pression de 1013 HPa à l'exclusion de l'air, il en est comme suit pour la distribution granulométrique: D(v, 0.9) (t_{21d}) : D(v, 0.9) (t_{0d}) < 2, de préférence < 1,5, de manière particulièrement préférée < 1,2,
ou
l'indice d'instabilité de l'émulsion H/E étant de 75 % ou moins, de préférence de 65 % ou moins, de manière particulièrement préférée de 55 % ou moins, par rapport à l'indice d'instabilité d'une émulsion H/E de comparaison qui, avec une composition par ailleurs identique, ne comprend pas d'hydroxyester de formule (I).

8. Préparation contenant un tensioactif, de préférence selon l'une quelconque des revendications 1 à 5, comprenant
- un ou plusieurs hydroxyester(s) de formule (I) tel(s) que défini(s) dans l'une quelconque des revendications 1 à 5, en tant qu'agent d'augmentation de la viscosité à 22,7 °C,
- un ou plusieurs tensioactif(s) anionique(s), de préférence choisi(s) dans le groupe constitué par les sulfates et les sulfonates, de manière particulièrement préférée comprenant le lauryl éther sulfate de sodium,
ainsi que, le cas échéant,
- un ou plusieurs autres tensioactif(s).

9. Utilisation
(a) d'un seul hydroxyester de formule (I) ou
(b) d'un mélange comprenant ou se composant de deux ou plusieurs hydroxyesters différents de formule (I), 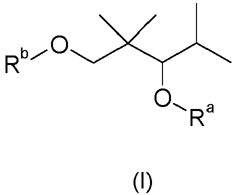 dans laquelle respectivement l'un des deux radicaux R^{a} ou R^{b} est l'hydrogène et l'autre radical R^{a} ou R^{b} respectif signifie un radical acyle à chaîne droite ou ramifié ayant de 2 à 10 atomes de carbone,
en tant qu'agent de modification d'une ou de plusieurs propriétés physiques d'une formulation, les propriétés physiques étant choisies dans le groupe constitué par la stabilité d'émulsion et la viscosité, de préférence en tant qu'agent d'augmentation de la stabilité d'émulsion et/ou de la viscosité d'une telle formulation.

10. Utilisation selon la revendication 9,
dans laquelle
- dans la variante (a) ledit un hydroxyester ou bien
- dans la variante (b) un, deux ou plusieurs des hydroxyesters
sont des hydroxyesters de formule (I) 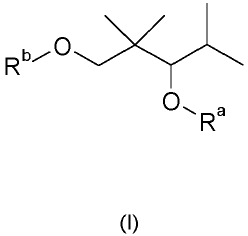 dans laquelle respectivement l'un des deux radicaux R^{a} ou R^{b} est l'hydrogène et l'autre radical R^{a} ou R^{b} respectif signifie un radical acyle à chaîne droite ou ramifié ayant de 2 à 6 atomes de carbone.

11. Utilisation selon la revendication 10, dans laquelle le radical acyle d'un, de deux, de plusieurs ou de tous les hydroxyesters de formule (I) est choisi dans le groupe constitué par l'acétyle, le propionyle, le n-butyryle, l'isobutyryle, le crotonyle, le n-pentanoyle, l'iso-pentanoyle, le n-hexanoyle et l'isohexanoyle,
de préférence choisi dans le groupe constitué par le n-butyryle, l'isobutyryle et le crotonyle.

12. Utilisation selon l'une quelconque des revendications précédentes 9 à 11,
- d'un hydroxyester de formule (I) tel que défini dans la revendication 1, 2 ou 3, dans lequel le radical R^{a} est l'hydrogène et le radical R^{b} signifie un radical acyle à chaîne droite ou ramifié
ou
- d'un mélange comprenant ou se composant
- d'un premier hydroxyester de formule (I) tel que défini dans la revendication 1, 2 ou 3, dans lequel le radical R^{a} est l'hydrogène et le radical R^{b} signifie un radical acyle à chaîne droite ou ramifié
ainsi que, en sus,
- d'un deuxième hydroxyester de formule (I) tel que défini dans la revendication 1, 2 ou 3.

13. Utilisation selon l'une quelconque des revendications précédentes 9 à 12,
- d'un mélange comprenant ou se composant
- d'un premier hydroxyester de formule (I) tel que défini dans la revendication 1, 2 ou 3, dans lequel le radical R^{a} est l'hydrogène et le radical R^{b} signifie un radical acyle à chaîne droite ou ramifié
et
- d'un deuxième hydroxyester de formule (I) tel que défini dans la revendication 1, 2 ou 3, dans lequel le radical R^{b} est l'hydrogène et le radical R^{a} signifie un radical acyle à chaîne droite ou ramifié,
dans lequel le radical R^{a} du premier hydroxyester a la signification de R^{b} du deuxième hydroxyester et le radical R^{b} du premier hydroxyester a la signification de R^{a} du deuxième hydroxyester.

14. Utilisation selon la revendication 12 ou 13,
d'un hydroxyester de formule (Ib) 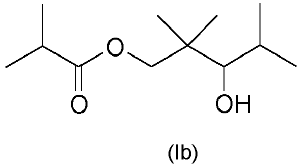 ou
d'un mélange comprenant ou se composant d'un premier et d'un deuxième hydroxyester de formule (I) tels que définis dans la revendication 1, 2 ou 3,
dans lequel le premier hydroxyester de formule (I) a la formule 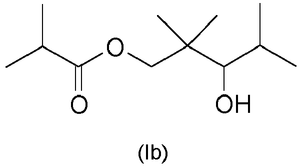 et le deuxième hydroxyester de formule (I) présente de préférence la formule

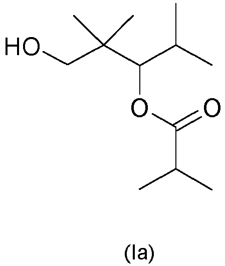

15. Procédé
(a) de production et/ou de stabilisation d'une émulsion H/E, comprenant les étapes suivantes consistant à:
- mélanger deux liquides en présence d'une quantité à effet émulsifiant d'un, de deux ou de plusieurs hydroxyester(s) de formule (I) tel(s) que défini(s) dans l'une quelconque des revendications 1 à 5
et/ou
(b) de production d'une préparation contenant un tensioactif et ayant une viscosité accrue, contenant un ou plusieurs tensioactif(s) anionique(s), comprenant les étapes suivantes consistant à:
- mélanger un liquide contenant un tensioactif, qui contient un ou plusieurs tensioactif(s) anionique(s), avec une quantité augmentant la viscosité, d'un, de deux ou de plusieurs hydroxyesters de formule (I) tel(s) que défini(s) dans l'une quelconque des revendications 1 à 5.
